(19) Europäisches Patentamt
European Patent Office
Office européen des brevets

(11) EP 3 271 473 B1

# (12) EUROPEAN PATENT SPECIFICATION

(45) Date of publication and mention of the grant of the patent:
**15.06.2022 Bulletin 2022/24**

(21) Application number: **16714658.8**

(22) Date of filing: **17.03.2016**

(51) International Patent Classification (IPC):
***C12Q 1/06*** *(2006.01)* ***G01N 33/543*** *(2006.01)*

(52) Cooperative Patent Classification (CPC):
**C12Q 1/06; G01N 33/54366;** G01N 2469/00

(86) International application number:
**PCT/US2016/022814**

(87) International publication number:
**WO 2016/149472 (22.09.2016 Gazette 2016/38)**

## (54) NONWOVEN ARTICLES FOR DETECTING MICROORGANISMS IN A FLUID SAMPLE AND METHODS OF USING THE NONWOVEN ARTICLES

NICHTGEWEBTE ARTIKEL ZUM NACHWEIS VON MIKROORGANISMEN IN EINER FLÜSSIGPROBE UND VERFAHREN ZUR VERWENDUNG VON NICHTGEWEBTEN ARTIKELN

ARTICLES NON TISSÉS PERMETTANT DE DÉTECTER DES MICROORGANISMES DANS UN ÉCHANTILLON DE FLUIDE ET PROCÉDÉS D’UTILISATION DES ARTICLES NON TISSÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.03.2015 US 201562135425 P**

(43) Date of publication of application:
**24.01.2018 Bulletin 2018/04**

(73) Proprietor: **3M Innovative Properties Company**
**St. Paul, MN 55133-3427 (US)**

(72) Inventors:
- **KSHIRSAGAR, Manjiri T.**
**Saint Paul, Minnesota 55133-3427 (US)**
- **RAJAGOPAL, Raj**
**Saint Paul, Minnesota 55133-3427 (US)**

(74) Representative: **Mathys & Squire**
**Theatinerstraße 7**
**80333 München (DE)**

(56) References cited:
**WO-A1-93/06924**
**WO-A1-2009/067518**
**WO-A1-2010/078284**
**WO-A1-2010/078404**
**WO-A2-2012/078374**
**WO-A2-2012/078426**
**US-A1- 2014 099 233**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field

The present disclosure relates to nonwoven articles and methods of using the nonwoven articles for detecting microorganisms in a fluid sample.

### Background

It is often desirable or necessary to assay for the presence of bacteria or other microorganisms in various clinical, food, environmental, or other samples, in order to determine the identity and/or the quantity of the microorganisms present. Bacterial DNA or bacterial RNA, for example, can be assayed to assess the presence or absence of a particular bacterial species even in the presence of other bacterial species. The ability to detect the presence of a particular bacterium, however, depends, at least in part, on the concentration of the bacterium in the sample being analyzed. Concentration of the bacteria in the sample can shorten the culturing time or even eliminate the need for a culturing step. Thus, methods have been developed to isolate (and thereby concentrate) particular bacterial strains by using antibodies specific to the strain (for example, in the form of antibody-coated magnetic or non-magnetic particles). Such methods, however, have tended to be expensive and still somewhat slower than desired for at least some diagnostic applications. Non-specific concentration or capture of microorganisms has been achieved through methods based upon carbohydrate and lectin protein interactions. Various inorganic materials (for example, hydroxyapatite and metal hydroxides) have also been used to non-specifically bind and concentrate bacteria. Such non-specific concentration methods have varied in speed, cost, sample requirements, space requirements, ease of use, suitability for on-site use, and/or effectiveness.

Bacteria occur naturally in most aquatic systems and can cause problems in industrial applications using water such as cooling towers, heat exchangers, oil and gas exploration, and hydraulic fracturing operations. The bacteria need to be monitored to develop and implement an effective biocide program to keep the bacteria within acceptable limits. The microbiological quality of water is typically assessed by traditional growth-based methods, which can take 24 to 48 hours to perform. Rapid methods based on ATP bioluminescence assays have been used to determine microbial contamination in water as they provide immediate results; however, the methods are limited by detection sensitivity because they require at least $1 \times 10^5$ colony forming units (cfu)/ml to elicit detectable responses. Interference from treatment chemicals can affect the bioluminescence, leading to erroneous results and mismanagement of biocides. One can increase the sensitivity of the ATP bioluminescence assay by using a larger volume of sample (e.g., 100 ml), but such methods can be difficult to implement in the field.

Patent application US2014099233 discloses a monitoring device comprising a test composition, a test element comprising a test portion to which the test composition is releasably adhered, a detection reagent, and a container comprising a first end with an opening and a second end opposite the first end. The test portion comprises fibrous material comprising nylon.

Patent application WO2012078374 discloses a process for capturing or concentrating microorganisms for detection or assay comprises (a) providing a concentration device comprising (1) a porous fibrous nonwoven matrix comprising nylon and (2) a plurality of particles of at least one concentration agent that comprises diatomaceous earth, the particles being enmeshed in the porous fibrous nonwoven matrix; (b) providing a sample comprising at least one target cellular analyte; (c) contacting the concentration device with the sample such that at least a portion of the at least one target cellular analyte is bound to or captured by the concentration device; and (d) detecting the presence of at least one bound target cellular analyte.

Patent application WO2012078426 discloses a process for capturing or concentrating microorganisms for detection or assay comprises (a) providing a concentration device comprising (1) a porous fibrous nonwoven matrix comprising nylon and (2) a plurality of particles of at least one concentration agent that comprises a metal silicate, the particles being enmeshed in the porous fibrous nonwoven matrix; (b) providing a sample comprising at least one target cellular analyte; (c) contacting the concentration device with the sample such that at least a portion of the at least one target cellular analyte is bound to or captured by the concentration device; and (d) detecting the presence of at least one bound target cellular analyte.Patent application WO2010078284 discloses a process comprises (a) providing (1) at least one sample suspected of comprising at least one coliform strain, (2) at least one culture device comprising at least one culture medium that is hydrated or hydratable, and (3) at least one particulate concentration agent that is substantially optically transparent when in contact with the culture medium in the culture device when the culture medium is hydrated; (b) placing the particulate concentration agent in contact with the sample such that at least a portion of the coliform strain is bound to the particulate concentration agent to form coliform-bound particulate concentration agent; (c) placing the coliform-bound particulate concentration agent in contact with the culture medium; (d) incubating the culture device comprising the coliform-bound particulate concentration agent in contact with the culture medium, the culture medium

being hydrated; and (e) optically detecting the presence of the coliform strain without separating the coliform strain from the particulate concentration agent.

Patent application WO2010078404 discloses a method for isolating microorganisms from a sample, the sample including sample matrix and microorganisms, the method including the steps of providing a receptacle, the receptacle configured to allow filtering of the sample and to reversibly contain the sample and a concentration agent; adding the sample to the receptacle, wherein a microorganism-bound composition will be formed in the receptacle, the microorganism-bound composition including concentration agent-bound microorganisms and sample matrix; and filtering the microorganism-bound composition through a filter to collect the concentration agent-bound microorganisms on the filter, wherein the filter has an average pore size that is greater than the average size of the microorganisms.

Patent application WO2009067518 discloses a system and method for preparing and analyzing samples. The system can include a sample preparation system and a sample detection system coupled to the sample preparation system. The sample preparation system can include a deformable self-supporting receptacle comprising a reservoir adapted to contain a liquid composition comprising a source and a diluent. The sample detection system can be positioned in fluid communication with the reservoir and can be adapted to analyze a sample of the liquid composition for an analyte of interest. The system can further include a fluid path defined at least partially by the reservoir and the sample detection system. The method can include applying pressure to the deformable self-supporting receptacle to move a sample of the liquid composition in the fluid path to the sample detection system and analyzing the sample for the analyte of interest with the sample detection system.

Patent application WO93/06924 discloses a particle loaded, porous, fibrous compressed or fused article comprises a nonwoven fibrous polymeric web, which may be a thermoplastic, melt-extrudable, and pressure-fusible blown microfibrous web, and sorptive particles enmeshed in said web, the particle loaded fibrous article has a Gurley number of at least two seconds. Patent application WO93/06924 also discloses a method of preparation of the article and a method of use thereof.

## Summary


Nonwoven articles are provided that can be used to detect microorganisms and/or cellular analytes in fluid samples, such as water or aqueous dispersions.

In a first aspect, a nonwoven article is provided according to the appended claims. The nonwoven article includes a) a fibrous porous matrix and b) a plurality of concentration agent particles enmeshed in the fibrous porous matrix. The fibrous porous matrix consists essentially of inorganic fibers and polymeric fibers.

In a second aspect, a method of detecting microorganisms and/or cellular analytes in a fluid sample is provided. The method includes a) providing a nonwoven article according to the first aspect and b) providing a fluid sample suspected of containing at least one microorganism strain or target cellular analyte. The method further includes c) contacting the fluid sample with the nonwoven article such that at least a portion of the at least one microorganism strain or target cellular analyte is bound to the nonwoven article and d) detecting the presence of the at least one bound microorganism strain or bound target cellular analyte.


## Brief Description of the Drawings

Figure 1 is a scanning electron microscope (SEM) image of the exemplary nonwoven article of Example 1.
Figure 2 is an SEM image of the exemplary nonwoven article of Example 2.
Figure 3 is an SEM image of the exemplary nonwoven article of Example 3.

## Detailed Description

Nonwoven articles and rapid methods for monitoring of microbial quality of fluid samples are provided. The method combines nonwoven articles that concentrate at least one microorganism or target cellular analyte and detection of the microorganism or target cellular analyte. The nonwoven articles may be contacted with large volumes of fluid samples to concentrate the microorganism and/or target cellular analyte, and also allow further optional washing to remove contaminants prior to detection. The nonwoven article is easily transferred to a receptacle for detection. Methods according to the disclosure are capable of readily detecting bacterial contamination in fluid samples in about 15 minutes. Accordingly, the nonwoven articles and methods can be suitable for field based detection of microorganisms and target cellular analytes in fluid samples.

For the following Glossary of defined terms, these definitions shall be applied for the entire application, unless a different definition is provided in the claims or elsewhere in the specification.

Glossary

Certain terms are used throughout the description and the claims that, while for the most part are well known, may require some explanation. It should be understood that, as used herein:
The term "a", "an", and "the" are used interchangeably with "at least one" to mean one or more of the elements being described.

The term "and/or" means either or both. For example "A and/or B" means only A, only B, or both A and B.

As used in this specification, the recitation of numerical ranges by endpoints includes all numbers subsumed within that range (e.g. 1 to 5 includes 1, 1.5, 2, 2.75, 3, 3.8, 4, and 5).

Unless otherwise indicated, all numbers expressing quantities or ingredients, measurement of properties and so forth used in the specification and embodiments are to be understood as being modified in all instances by the term "about." Accordingly, unless indicated to the contrary, the numerical parameters set forth in the foregoing specification and attached listing of embodiments can vary depending upon the desired properties sought to be obtained by those skilled in the art utilizing the teachings of the present disclosure. At the very least, and not as an attempt to limit the application of the doctrine of equivalents to the scope of the claimed embodiments, each numerical parameter should at least be construed in light of the number of reported significant digits and by applying ordinary rounding techniques.

The term "comprises" and variations thereof do not have a limiting meaning where these terms appear in the description and claims.

The term "consists essentially of' does not exclude the presence of additional materials which do not significantly affect the desired characteristics of a given composition or product.

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure.

The term "cannulated device" means a device that includes a tube, such as a narrow flexible tube.

The term "cellular analyte" means an analyte of cellular origin (that is, a microorganism or a component thereof (for example, a cell or a cellular component such as deoxyribonucleic acid (DNA) or ribonucleic acid (RNA), proteins, nucleotides such as adenosine triphosphate (ATP), and the like, and combinations thereof); references to a microorganism or microorganism strain throughout this specification are meant to apply more generally to any cellular analyte).

The term "concentration agent" means a material or composition that binds microorganisms and/or cellular analytes from a fluid sample (preferably, having a cellular analyte capture or binding efficiency of at least about 60 percent, or at least about 70 percent, or at least about 80 percent, or at least about 90 percent), thereby concentrating the microorganisms and/or cellular analytes into a smaller volume than when present in the fluid sample.

The term "detection" means the identification of a cellular analyte (for example, at least a component of a target microorganism, which thereby determines that the target microorganism is present).

The term "enmeshed" (in regard to particles in a fibrous porous matrix) means that the particles are entrapped in and on the fibrous porous matrix (and, preferably, distributed within it), rather than solely being borne on its surface.

The term "fibrillated" (in regard to fibers or fibrous material) means treated (for example, by beating) in a manner that forms fibrils or branches attached to a fiber's main trunk.

The term "fibrous porous matrix" means a nonwoven web or medium, (i.e., not a woven or knitted fabric), comprising interlaid fibers, for example, a web comprising fibers that are interlaid by meltblowing, spunbonding, or other air laying techniques; carding; wet laying; or the like. Typically, the fibers have lengths of less than 100 millimeters and are uncrimped.

The term "filtering" is generally used to describe the process of separating matter by size, charge and/or function. For example, filtering can include separating soluble matter and a solvent (e.g., diluent) from insoluble matter, or it can include separating soluble matter, a solvent and relatively small insoluble matter from relatively large insoluble matter. A variety of filtration methods can be used, including, but not limited to, passing the liquid composition through a filter, settling followed by aspiration or decanting, other suitable filtration methods, and combinations thereof. "Settling" is used to refer to allowing the insoluble matter in the liquid composition to settle. Settling may occur by gravity or by centrifugation. The insoluble matter (or relatively large insoluble matter) can then be separated from the soluble matter (or soluble matter and relatively small insoluble matter) and solvent by aspirating the soluble matter and solvent from the insoluble matter, decanting the soluble matter and solvent, or a combination thereof.

The term "filtrate" is generally used to describe the liquid remaining after the insoluble matter (or at least the relatively large insoluble matter) has been removed from the liquid composition.

The term "fluid" means liquid, solution, or dispersion of solid or liquid in liquid.

The term "lumened device" means a device that includes a tubular interior or exterior shape.

The term "microorganism" means any cell or particle having genetic material suitable for analysis or detection (including, for example, bacteria, yeasts, viruses, and bacterial endospores).

The term "microorganism strain" means a particular type of microorganism that is distinguishable through a detection method (for example, microorganisms of different genera, of different species within a genera, or of different isolates within a species).

The terms "polymer" and "polymeric material" are used interchangeably and refer to materials formed by reacting one or more monomers.

The term "sample" means a substance or material that is collected (for example, to be analyzed).

The term "sample matrix" means the components of a sample other than microorganisms and/or cellular analytes.

The term "target cellular analyte" means any cellular analyte that is desired to be detected.

The term "target microorganism" means any microorganism that is desired to be detected.

Reference throughout this specification to "one embodiment," "certain embodiments," "one or more embodiments" or "an embodiment," whether or not including the term "exemplary" preceding the term "embodiment," means that a particular feature, structure, material, or characteristic described in connection with the embodiment is included in at least one embodiment of the certain exemplary embodiments of the present disclosure. Thus, the appearances of the phrases such as "in one or more embodiments," "in some embodiments," "in certain embodiments," "in one embodiment," "in many embodiments" or "in an embodiment" in various places throughout this specification are not necessarily referring to the same embodiment of the certain exemplary embodiments of the present disclosure. Furthermore, the particular features, structures, materials, or characteristics may be combined in any suitable manner in one or more embodiments.

Various exemplary embodiments of the disclosure will now be described. Exemplary embodiments of the present disclosure may take on various modifications and alterations without departing from the scope of the appended claims. Accordingly, it is to be understood that the embodiments of the present disclosure are not to be limited to the following described exemplary embodiments, but are to be controlled by the limitations set forth in the claims.

In a first aspect, a nonwoven article is provided. The nonwoven article includes a) a fibrous porous matrix and b) a plurality of concentration agent particles enmeshed in the fibrous porous matrix. The fibrous porous matrix consists essentially of inorganic fibers and polymeric fibers wherein the inorganic fibers comprise glass fibers, wherein the polymeric fibers comprise fibrillated polyethylene fibers and bi-component fibers, wherein the fibrous porous matrix is free of polyamide fibers and wherein the fibrous porous matrix is a nonwoven fibrous layer comprising inorganic fibers and uncrimped polymeric fiber and wherein the concentration agent particles comprise amorphous metal silicates, guanidine-functionalized metal silicates, diatomaceous earth, surface-modified diatomaceous earth, gamma-FeO(OH), metal carbonates, metal phosphates, silica, perlite, guanidine-functionalized perlite, guanidine-functionalized diatomaceous earth, or a combination thereof. The nonwoven fibrous porous matrix is often in the form of a layer of interlaid fibers that are not woven or knitted together. The nonwoven, fibrous porous matrix can be prepared by any suitable process such as, for example, air laying techniques, spunlaid techniques such as meltblowing or spunbonding, carding, wetlaying, and combinations thereof. In many embodiments, the fibrous nonwoven matrix is prepared by wetlaid techniques.

Fibers suitable for use in preparing the nonwoven fibrous porous matrix are usually pulpable or extrudable fibers such as those that are stable to radiation and/or to a variety of solvents. Optionally, at least some of the polymeric fibers can be selected to exhibit a degree of hydrophilicity. Useful fibers include polymeric fibers, inorganic fibers, and combinations thereof. More particularly, the fibers include a plurality of different types of fibers, including polyolefin fibers and fiberglass fibers.

Suitable polymeric fibers include those made from natural polymers (those derived from animal or vegetable sources) and/or synthetic polymers, including thermoplastic and solvent-dispersible polymers. Useful polymers include polyolefins (for example, poly(ethylene) (e.g., low density polyethylene, medium density polyethylene, high density polyethylene, etc.), polypropylene, poly(1-butene), copolymers of ethylene and propylene, alpha olefin copolymers such as copolymers of ethylene or propylene with 1-butene, 1-hexene, 1-octene, and 1-decene such as poly(ethylene-co-1-butene), poly(ethylene-co-1-butene-co-1-hexene), and the like); polylactic acid; poly(isoprenes); poly(butadienes); polyimides (for example, poly(pyromellitimide) and the like); polyethers; poly(ether sulfones) (for example, poly(diphenylether sulfone), poly(diphenylsulfone-co-diphenylene oxide sulfone), and the like); poly(sulfones); poly(vinyl esters) such as poly(vinyl acetates); copolymers of vinyl acetate (for example, poly(ethylene-co-vinyl acetate), copolymers in which at least some of the acetate groups have been hydrolyzed to provide various poly(vinyl alcohols) including poly(ethylene-co-vinyl alcohol), and the like); poly(phosphazenes); poly(vinyl ethers); poly(vinyl alcohols); polyaramids (for example, paraaramids such as poly(paraphenylene terephthalamide) and fibers sold under the trade designation "KEVLAR" by DuPont Co., Wilmington, DE, pulps of which are commercially available in various grades based on the length of the fibers that make up the pulp such as, for example, "KEVLAR 1F306" and "KEVLAR 1F694", both of which include aramid fibers that are at least 4 mm in length; and the like); wool; silk; cellulosic polymers (for example, cellulose, cellulose derivatives such as rayon, and the like); acrylic polymers (for example, polyacrylonitrile); polyesters (for example, polyethylene terephthalate); fluorinated polymers (for example, poly(vinyl fluoride), poly(vinylidene fluoride), copolymers of

vinylidene fluoride such as poly(vinylidene fluoride-co-hexafluoropropylene), copolymers of chlorotrifluoroethylene such as poly(ethylene-co-chlorotrifluoroethylene), and the like); chlorinated polymers; poly(carbonates); and the like; and combinations thereof. In certain embodiments, the polymeric fibers comprise a polyolefin, a polysulfone, or a combination thereof.

Inorganic fibers include those that contain at least one inorganic material selected from glasses. These fibers are often added to provide strength to the fibrous porous matrix. For example, porous matrix layers containing inorganic fibers are often capable of being bent, folded, or pleated without breaking apart. In general,inorganic fibers include, for example, fiberglass (for example, E-glass, S-glass, and the like), ceramic fibers (for example, fibers made of metal oxides (such as alumina), silicon carbide, boron nitride, boron carbide, and the like), and combinations thereof. Ceramic fibers can be at least partially crystalline (exhibiting a discernible X-ray powder diffraction pattern or containing both crystalline and amorphous (glass) phases). The inorganic fibers include fiberglass.

To facilitate entrapment of the concentration agent particles and/or to ensure a high surface area, the fibers used to form the nonwoven, fibrous porous matrix contain at least one fibrillated fiber (for example, in the form of a main fiber surrounded by many smaller attached fibrils). The main fiber generally can have a length in the range of 0.5 millimeters to 5 millimeters and a diameter in a range of 1 micrometer to 20 micrometers. The fibrils typically can have a submicrometer diameter. In many embodiments, the fibrillated fibers are prepared from a polyolefin such as poly(ethylene) or polypropylene, or from an acrylic polymer such as polyacrylonitrile.

Suitable polymeric fibers further include bi-component fibers, which typically assist in binding all of the matrix fibers together due to a difference in melting point of one of the materials in the bi-component fiber. Bi-component fibers can have, for example, a core-sheath structure, a side-by-side structure, an islands-in-the-sea structure, or a segmented-pie structure, or the like. An example side-by-side bi-component fiber is the polyolefin thermally bonded bi-component fiber that is commercially available from Chisso Corporation (Osaka, Japan) under the trade designation CHISSO (for example, CHISSO ES). An example core-sheath bi-component fiber is commercially available from Unitika Ltd. (Osaka, Japan) under the trade designation MELTY (for example, MELTY 4080) and those commercially available from Minifibers, Inc. (Johnson City, TN) made of ethyl vinyl acetate (sheath) and polypropylene (core), or made of a co-polyester of polyester and polyethylene terephthalate (PET) (sheath) and polyester (core).

The nonwoven fibrous porous matrix contains a plurality of different types of fibers. In some embodiments, the porous matrix can be formed using three, four, or even more different types of fibers. For example, a fiberglass fiber can be added for strength and integrity, while fibrillated poly(ethylene) can be added for entrapment of the particulates. Additionally, nylon fibers (not comprised in the fibrous porous matrix of the invention) provide hydrophilic character while fibrillated poly(ethylene) fibers provide hydrophobic character to the porous matrix. If fibrillated and non-fibrillated fibers are used in combination, the weight ratio of fibrillated fibers to non-fibrillated fibers is often at least 1:2, at least 1:1, at least 2:1, at least 3:1, at least 5:1, or even at least 8:1. In some embodiments, mixtures of hydrophobic and hydrophilic polymeric fibers are used. For example, the fibrous porous matrix can include a mixture of hydrophobic fibers such as polyolefins plus hydrophilic fibers such as polysulfones. In some specific examples, the polymeric fibers include polyolefin fibers, bi-component fibers, and fiberglass fibers.

The fibrous porous matrix is free of polyamide fibers. It has been discovered that the inclusion of nylon fibers in the fibrous porous matrix can result in lower luminescence than the fibrous porous matrix without the nylon fibers for a bioluminescent ATP detection method, as discussed in the Examples below.

The fibers used to form the nonwoven fibrous porous matrix are uncrimped. In contrast to uncrimped fibers, crimped fibers may be identified as displaying repeating features (as manifested e.g., in a wavy, jagged, sinusoidal, etc., appearance of the fiber), by having a helical appearance (e.g., particularly in the case of crimped fibers obtained by thermal activation of bi-component fibers), and the like, and are readily recognizable by those of ordinary skill in the art. Exemplary crimped fibers are described in U.S. Pat. No. 4,118,531 to Hauser and U.S. Pat. No. 5,597,645 to Pike et al., and CA Patent 2612854 to Sommer et al.

The fibers used to form the nonwoven fibrous porous matrix can be of a length and diameter that can provide a porous matrix having sufficient structural integrity and sufficient porosity for a particular application (for example, passing a fluid sample through the matrix). The fiber lengths are often at least about 0.5 millimeter, at least 1 millimeter, at least 2 millimeters, at least 3 millimeters, at least 4 millimeters, at least 6 millimeters, at least 8 millimeters, at least 10 millimeters, at least 15 millimeters, or at least 20 millimeters, and up to 50 millimeters, up to 40 millimeters, up to 30 millimeters, or up to 25 millimeters. The diameter of the fibers can be, for example, at least 10 micrometers, at least 20 micrometers, or at least 30 micrometers. The fiber lengths and diameters will vary depending upon factors such as the nature of the fiber and the type of application.

The nonwoven fibrous porous matrix often includes a mixture of polyolefin fibers, glass fibers, and bi-component fibers. In some particular embodiments, the nonwoven fibrous porous matrix contains a mixture of fibrillated polyethylene fibers, glass fibers, and sheath-core bi-component fibers. In some examples, the nonwoven fibrous porous matrix contains 40 to 80 weight percent fibrillated polyethylene fibers, 5 to 20 weight percent glass fibers, and 5 to 20 weight percent bi-component fibers. In some examples (not of the invention) , the nonwoven fibrous porous matrixcontains 40 to 80

weight percent fibrillated polyethylene fibers, 10 to 30 weight percent nylon fibers, 5 to 20 weight percent glass fibers, and 5 to 20 weight percent bi-component fibers. In other examples, the nonwoven, fibrous porous matrix contains 50 to 70 weight percent fibrillated polyethylene fibers, 5 to 15 weight percent glass fibers, and 5 to 20 weight percent bi-component fibers. In still other examples (not of the invention), the fibrous porous matrix contains 55 to 65 weight percent fibrillated polyethylene fibers, 0 to 20 weight percent nylon fibers, 5 to 15 weight percent glass fibers, and 10 to 20 weight percent bi-component fibers.

As noted above, the fibrous porous matrix consists essentially of inorganic fibers and polymeric fibers. Accordingly, in most embodiments, the fibrous porous matrix contains only fibers. For example, at least 90 weight percent, at least 95 weight percent, at least 98 weight percent, at least 99 weight percent, or at least 99.5 weight percent of a dry fibrous porous matrix is fibers. In certain embodiments, the nonwoven article comprises a thickness of at least 0.1 millimeters, or at least 0.15 millimeters, or at least 0.2 millimeters, or at least 0.3 millimeters, or at least 0.4 millimeters, or at least 0.5 millimeters, or at least 0.6 millimeters. The nonwoven article usually comprises a thickness of up to 1 millimeter, or up to 0.9 millimeters, or up to 0.8 millimeters, or up to 0.7 millimeters, or up to 0.55 millimeters. Stated differently, the nonwoven article may comprise a thickness of between 0.15 millimeters and 1 millimeter, or between 0.15 millimeters and 0.8 millimeters, or between 0.1 millimeters and 0.7 millimeters. In certain embodiments, a nonwoven article having a thickness towards the lower end of the thickness range is selected to minimize interference with detection of the microorganisms and/or cellular analytes, such as decreasing time required for a reagent to diffuse into the nonwoven article, or decreasing blockage of a generated detection signal.

The nonwoven article typically includes both the fibrous porous matrix and concentration agent particles distributed within the fibrous porous matrix. In most embodiments, the nonwoven article contains at least 10 weight percent concentration agent particles based on a total dry weight of the nonwoven article. If the amount of the concentration agent particles is lower than about 10 weight percent, the nonwoven article may not contain enough concentration agent particles to effectively capture microorganisms or cellular analytes from a fluid sample. In some examples, the nonwoven article contains at least 15 weight percent, at least 20 weight percent, at least 25 weight percent, or at least 30 weight percent concentration agent particles based on a total dry weight of the nonwoven article.

On the other hand, the nonwoven article usually contains no greater than 55 weight percent concentration agent particles based on the total dry weight of the nonwoven article. If the amount of the concentration agent particles is greater than about 55 weight percent, the nonwoven article may contain an insufficient amount of the fibrous porous matrix. That is, the strength of the nonwoven article may be insufficient to hold together when employed to capture microorganism strains and/or target cellular analytes. In some examples, the nonwoven article contains no greater than 50 weight percent, no greater than 45 weight percent, or no greater than 40 weight percent concentration agent particles based on a total weight of the nonwoven article.

Stated differently, the nonwoven article often contains 10 to 55 weight percent concentration agent particles and 45 to 90 weight percent fibrous porous matrix, 15 to 50 weight percent concentration agent particles and 50 to 85 weight percent fibrous porous matrix, 20 to 50 weight percent concentration agent particles and 50 to 80 weight percent fibrous porous matrix, 20 to 45 weight percent concentration agent particles and 55 to 80 weight percent fibrous porous matrix, 25 to 40 weight percent concentration agent particles and 60 to 75 weight percent fibrous porous matrix, or 30 to 40 weight percent concentration agent particles and 60 to 70 weight percent fibrous porous matrix. The amounts are based on the total dry weight of the nonwoven article.

In many embodiments, the nonwoven article (when dry) contains only concentration agent particles and fibrous porous matrix. For example, the nonwoven article contains at least 90 weight percent, at least 95 weight percent, at least 98 weight percent, at least 99 weight percent, or at least 99.5 weight percent combined concentration agent particles and fibrous porous matrix when dry.

Concentration agent particles are water-insoluble particulate materials that have been employed to non-specifically capture microorganism strains, cellular analytes, or a combination thereof, when contacted with fluid samples containing microorganisms and/or cellular analytes. The concentration agent particles typically comprise microparticles. The concentration agent particles typically comprise particles selected from the group consisting of amorphous metal silicates, guanidine-functionalized metal silicates, diatomaceous earth, surface-modified diatomaceous earth, guanidine-functionalized diatomaceous earth, gamma-FeO(OH), metal carbonates, metal phosphates, silica, perlite, guanidine-functionalized perlite, and combinations thereof.

In an embodiment, the concentration agent particles comprise particles of amorphous metal silicates, such as amorphous, spheroidized magnesium silicate, amorphous, spherical aluminum silicate, or a combination thereof. Amorphous, at least partially fused particulate forms of metal silicate can be prepared by any of the known methods of melting or softening relatively small feed particles (for example, average particle sizes up to about 25 micrometers) under controlled conditions to make generally ellipsoidal or spheroidal particles (that is, particles having magnified two-dimensional images that are generally rounded and free of sharp corners or edges, including truly or substantially circular and elliptical shapes and any other rounded or curved shapes). Such methods include atomization, fire polishing, direct fusion, and the like. A preferred method is flame fusion, in which at least partially fused, substantially glassy particles

are formed by direct fusion or fire polishing of solid feed particles (for example, as in the method described in U.S. Patent No. 6,045,913 (Castle et al.). Most preferably, such methods can be utilized to produce amorphous, spheroidized metal silicates by converting a substantial portion of irregularly-shaped feed particles (for example, from about 15 to about 99 volume percent; preferably, from about 50 to about 99 volume percent; more preferably, from about 75 to about 99 volume percent; most preferably, from about 90 to about 99 volume percent) to generally ellipsoidal or spheroidal particles.

Some amorphous metal silicates are commercially available. For example, amorphous, spheroidized magnesium silicate was commercially available for use in cosmetic formulations (for example, "3M COSMETIC MICROSPHERES CM-111", available from 3M Company, St. Paul, MN). 3M COSMETIC MICROSPHERES CM-111 have a particle density of 2.3 g/cc, a surface area of 3.3 $m^2/g$, and have a particle size of: 90 percent less than 11 microns (i.e., $D_{90}$ = 11), 50 percent less than 5 microns, and 10 percent less than 2 microns. Amorphous, spherical aluminum silicate is commercially available for use in paints, primers, powder coatings, and other coatings, for example, "3M CERAMIC MICROSPHERES" from 3M Company, St. Paul, MN. The 3M CERAMIC MICROSPHERES are alkali alumino silicate ceramic microspheres shaped as solid spheres with particle density of 2.4 g/cc, and are commercially available in three grades: W-210, W-410, and W0610. W-210 particles have a surface area of 5 $m^2$/cc and a particle size of: 95 percent less than about 12 microns (i.e., $D_{95}$ = 12), 90 percent less than about 9 microns, 50 percent less than about 3 microns, and 10 percent less than about 1 micron. W-410 particles have a surface area of 3 $m^2$/cc and a particle size of: 95 percent less than about 24 microns (i.e., $D_{95}$ = 24), 90 percent less than about 15 microns, 50 percent less than about 4 microns, and 10 percent less than about 1 micron. W-610 particles have a surface area of 3 $m^2$/cc and a particle size of: 95 percent less than about 40 microns (i.e., $D_{95}$ = 40), 90 percent less than about 28 microns, 50 percent less than about 10 microns, and 10 percent less than about 1 micron.

In certain embodiments, the concentration agent particles comprise perlite particles. Perlite is a naturally-forming amorphous volcanic glass, containing about 70-75% silicon dioxide and 12-15% aluminum oxide, as well as smaller amounts of other metal oxides, including sodium oxide, potassium oxide, iron oxide, magnesium oxide, and calcium oxide. When perlite is expanded by heat it forms a lightweight aggregate. Examples of suitable perlite particles include the 4106 grade material, the 4156 grade material, and the 476 grade material, all commercially available from Dicaperl Minerals Corporation (Crawfordsville, IN).

In certain embodiments, the concentration agent particles comprise guanidine-functionalized metal silicate particles, guanidine-functionalized diatomaceous earth particles, or guanidine-functionalized perlite particles. A guanidine-functionalized particle can be made, for example, according to methods disclosed in commonly assigned International Application No. PCT/US2014/040861 (Kshirsagar et al.). A guanidine-functionalized metal silicate particle, guanidine-functionalized diatomaceous earth particle, or guanidine-functionalized perlite particle comprises at least one guanidine-containing ligand. The guanidine-containing ligand is formed by modifying the metal silicate or perlite particle with a guanidine-containing silane having the structure shown in Formula 1:

$$X_{3-n}R^a{}_n\text{Si-Y-G} \qquad \text{Formula 1}$$

In Formula 1, Si is a silicon atom, and G denotes a guanidine group of the formula $-NH-C(=NH)-NH_2$. Y is a divalent group that is covalently bonded to the silicon atom at one end and to the G group at the other end. Each $R^a$ group, if any are present, is independently an alkyl, aralkyl, or aryl group, and is attached to the silicon atom. Each X is a leaving group covalently bonded to the silicon atom and is independently alkoxy or acyloxy, and *n* is 0, 1, or 2. A typical alkylene can be up to 20, up to 16, 12, 10, 8, 7, 6, 5, 4, or even up to 3 carbons, or even 2 carbons, inclusive of the terminal atoms of the divalent group. In some embodiments, Y is a divalent group comprising an alkylene of 3 to 6 carbons. In a preferred embodiment, Y is a divalent group having 3 carbons (i.e., propyl).

In Formula 1, each leaving group X is independently an alkoxy group of 1, 2, 3, 4, 5, 6, 7, 8, 9, or even up to 10 carbons, or is an acyloxy group of 2 carbons, or 3, 4, 5, 6, 7, 8, 9, or even up to 10 carbons, where the alkoxy or acyloxy group is bonded to the silicon through an oxygen atom.

In some embodiments, *n* is 0. When *n* is 0, no $R^a$ groups are present, and Formula 1 can be re-written more simply as shown in Formula 2 (where Si, G, Y, and X are as defined for Formula 1):

$$X_3\text{Si-Y-G} \qquad \text{Formula 2}$$

When the silane of Formula 1 (or Formula 2) reacts with an -OH group on the surface of a metal silicate, diatomaceous earth, or perlite particle, at least one X leaving group is replaced by a covalent bond of between the silicon atom and an oxygen atom on the surface of the metal silicate or perlite particle. An embodiment of a guanidine-functionalized metal silicate, diatomaceous earth or perlite particle comprising a specific exemplary guanidine-containing ligand within the general type represented by Formula 1, wherein *n* = 0 (i.e., as in Formula 2), is shown in Formula 3 (the circle in Formula 3 represents a metal silicate or perlite particle):

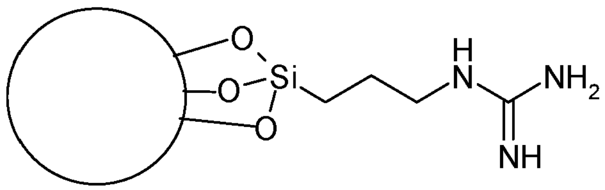

Formula 3

It will be understood that Formula 3 represents a specific embodiment wherein *n* is 3 and Y is a divalent group that is alkylene having 3 carbons. In each of Formulas 1 to 3, the ionization state of the guanidine group is omitted; however, it will be understood that in various environments such guanidine groups may be charged or uncharged (e.g., protonated or deprotonated), for example, according to the pH of a liquid medium in which the guanidine group is present.

The covalent bond(s) between the oxygen(s) of the ligand and the particle can be conveniently obtained, for example, by reacting a Si-bonded hydrolyzable group of the guanidine-containing precursor with a hydroxyl group of the particle. While the exemplary structure of Formula 3 shows three such bonded oxygen atoms (i.e., *n* = 3 in Formula 1), it will be appreciated that in various embodiments one, two or three such bonded oxygen atoms can be provided. If less than three such oxygen atoms are bonded to the silicon atom, other substituents (e.g., substituents that are not bonded to the particle, and which are not shown in Formula 1) may be present on the silicon atom. For example, the guanidine-containing ligand can include a polymeric structure involving formation of Si-O-Si (i.e., siloxane) groups, resulting from Si-O bonds being formed between two or more guanidine-containing ligand precursors. Without being bound by theory, it is thought that Si-O-Si groups may form in the presence of added water, or other aqueous solvents, or other agent that can hydrolyze bonds in Si-O-R groups, to give rise to more complex guanidine-containing ligand structures attached to particles.

A network of polymerized guanidine-containing ligands can form a coating on the surface of the metal silicate, diatomaceous earth, or perlite particle. In some embodiments it may be desirable to obtain the particle functionalized with polymerized guanidine-containing ligand (e.g., having at least one Si-O-Si group in the polymerized guanidine-containing ligand), as a means of increasing the loading of nitrogen-containing guanidine groups on the surface of the metal silicate, diatomaceous earth, or perlite particle. It is thought that in at least these types of polymerizations, a loading of nitrogen-containing guanidine groups on the surface of the metal silicate, diatomaceous earth, or perlite particle can attain levels of surface nitrogen content in a range from 1 to 10 atomic percent, as can be measured, for example, by X-ray photoelectron spectroscopy.

Guanidine-functionalized particles of the present disclosure include metal silicate particles, diatomaceous earth particles, and perlite particles. Useful metal silicates include silicates of metals such as magnesium, calcium, zinc, aluminum, iron, titanium, and the like (preferably, magnesium and aluminum), and combinations thereof. Preferred are amorphous metal silicates in at least partially fused particulate form. In certain embodiments, more preferred are amorphous, spheroidized metal silicates; and even more preferably, amorphous, spheroidized magnesium silicate. In certain embodiments, more preferred are amorphous aluminum silicates. Metal silicates are known and can be chemically synthesized by known methods or obtained through the mining and processing of raw ores that are naturally-occurring. The metal silicate particle, such as a magnesium silicate particle, bears sufficient surface hydroxyl groups (typically, Si-OH groups) to enable a desired number of guanidine-containing ligands to be covalently attached thereto. Useful perlites include the 4106, 4156, and 476 grade materials from Dicaperl Minerals Corporation (Crawfordsville, IN). Useful diatomaceous earth particles can be obtained from natural sources, and are commercially available from Alfa Aesar (A Johnson Matthey Company, Ward Hill, MA) or Dicaperl Minerals Corporation (Crawfordsville, IN).

The guanidine-functionalized metal silicate, diatomaceous earth, or perlite particles used in nonwoven articles of the present disclosure can be used in essentially any particulate form (preferably, a relatively dry or volatiles-free form) that is amenable to blending with fibers to form the nonwoven articles of the present disclosure. Preferably, the guanidine-functionalized particles are used in the form of a powder. Useful powders include those that comprise microparticles (preferably, microparticles having a particle size in the range of about 1 micrometer (more preferably, about 2 micrometers; even more preferably, about 3 micrometers; most preferably, about 4 micrometers) to about 100 micrometers (more preferably, about 50 micrometers; even more preferably, about 25 micrometers; most preferably, about 15 or 20 micrometers; where any lower limit can be paired with any upper limit of the range, as referenced above).

XPS is a technique that can provide information about the elemental and chemical (oxidation state and/or functional group) concentrations present on a solid surface. XPS typically provides an analysis of the outermost 3 to 10 nanometers (nm) of the specimen surface. XPS is sensitive to all elements in the periodic table except hydrogen and helium with detection limits for most species in the 0.1 to 1 atomic percent concentration range. In some cases, for example for CM-111 particles, a preferred surface composition assessment conditions for XPS can include a take-off angle of 90 degrees measured with respect to the sample surface with a solid angle of acceptance of ± 10 degrees. A person skilled in the art can select a suitable instrument setting for analysis of particles of the present disclosure.

In embodiments of the present disclosure, guanidine-functionalized metal silicate particles have a surface nitrogen content in a range from 1 atomic percent to 20 atomic percent, as measured by XPS. In some embodiments, the guanidine-functionalized metal silicate particles have a surface nitrogen content of at least 1 atomic percent, at least 2, at least 3, at least 4, or even at least 5 atomic percent, as measured by XPS. In some embodiments, the guanidine-functionalized metal silicate particles have a surface nitrogen content of up to 20 atomic percent, up to 15, up to 10, up to 9, up to 8, up to 7, or even up to 6 atomic percent, as measured by XPS. The surface nitrogen content of the guanidine-functionalized metal silicate particles, as measured by XPS, may be any combination of these lower and upper values, inclusive of the values thereof. A person skilled in the art would understand that in some embodiments it may be preferred to select higher or lower surface nitrogen content within these ranges, depending on the desired application. Suitable guanidine-functionalized perlite particles for use according to the present disclosure include those that comprise perlite and that have a surface nitrogen content of greater than 2 and less than or equal to about 12, as determined by XPS. Suitable guanidine-functionalized diatomaceous earth particles for use according to the present disclosure include those that comprise diatomaceous earth and have a surface composition having surface nitrogen content of greater than 2 and less than or equal to about 12.

In some embodiments, particularly preferred are guanidine-functionalized magnesium silicate particles. Suitable guanidine-functionalized magnesium silicate particles for use according to the present disclosure include those that comprise an amorphous magnesium silicate and that have a surface composition having a metal atom to silicon atom ratio greater than 0.01 and less than or equal to about 0.5 (preferably, less than or equal to about 0.4; more preferably, less than or equal to about 0.3; most preferably, less than or equal to about 0.2), as determined by X-ray photoelectron spectroscopy ("XPS", also known as Electron Spectroscopy for Chemical Analysis ("ESCA")). In some embodiments, particularly preferred are guanidine-functionalized aluminum silicate particles. Suitable guanidine-functionalized aluminum silicate particles for use according to the present disclosure include those that comprise an amorphous aluminum silicate and that have a surface composition having a metal atom to silicon atom ratio greater than 6.7 and less than or equal to about 17.3, as determined by XPS (also known as ESCA). In some embodiments, particularly preferred are guanidine-functionalized perlite particles.

In an embodiment, the concentration agent particles comprise particles of diatomaceous earth, for instance particles of surface-modified diatomaceous earth. Diatomaceous earth (or kieselguhr) is a natural siliceous material produced from the remnants of diatoms, a class of ocean-dwelling microorganisms. Thus, it can be obtained from natural sources and is also commercially available (for example, from Alfa Aesar, A Johnson Matthey Company, Ward Hill, MA). Diatomaceous earth particles generally comprise small, open networks of silica in the form of symmetrical cubes, cylinders, spheres, plates, rectangular boxes, and the like. The pore structures in these particles can generally be remarkably uniform.

Diatomaceous earth can be used in carrying out the process of the invention as the raw, mined material or as purified and optionally milled particles. Preferably, the diatomaceous earth is in the form of milled particles with sizes in the range of about 1 micrometer to about 50 micrometers in diameter (more preferably, about 3 micrometers to about 10 micrometers). The diatomaceous earth can optionally be heat treated prior to use to remove any vestiges of organic residues. If a heat treatment is used, it can be preferable that the heat treatment be at 500°C or lower, as higher temperatures can produce undesirably high levels of crystalline silica.

Surface-modified diatomaceous earth comprises diatomaceous earth bearing, on at least a portion of its surface, a surface treatment comprising titanium dioxide, ferric oxide, fine-nanoscale gold or platinum, or a combination thereof. Useful surface modifiers include fine-nanoscale gold; fine-nanoscale platinum; fine-nanoscale gold in combination with at least one metal oxide (preferably, titanium dioxide, ferric oxide, or a combination thereof); titanium dioxide; titanium dioxide in combination with at least one other (that is, other than titanium dioxide) metal oxide; and the like; and combinations thereof. Preferred surface modifiers include fine-nanoscale gold; fine-nanoscale platinum; fine-nanoscale gold in combination with at least ferric oxide or titanium dioxide; titanium dioxide; titanium dioxide in combination with at least ferric oxide; and combinations thereof. Surface-modified diatomaceous earth can be made, for example, according to methods disclosed in commonly assigned International Publication No. WO 2009/046191 (Kshirsagar et al.).

In an embodiment, the concentration agent particles comprise particles of gamma-FeO(OH) (also known as lepidocrocite). Specific examples of such concentration agent particles are disclosed in commonly assigned International Publication No. WO2009/046183 (Kshirsagar et al.). Gamma-FeO(OH) particles have been found to be surprisingly more effective than other iron-containing concentration agent particles in capturing gram-negative bacteria, which can be of great concern in regard to human bacterial infections.

Gamma-FeO(OH) is known and can be chemically synthesized by known methods (for example, by oxidation of ferrous hydroxide at neutral or slightly acidic pHs, as described for purposes of magnetic tape production in U.S. Pat. No. 4,729,846 (Matsui et al.). Gamma-FeO(OH) is also commercially available (for example, from Alfa Aesar, A Johnson Matthey Company, Ward Hill, Mass., and from Sigma-Aldrich Corporation, St. Louis, Mo.).

In an embodiment, the concentration agent particles comprise particles of silica. A specific example of concentration agent silica particles is silicon dioxide microspheres having a mean diameter of about 2.5 microns that are

commercially available from PolySciences, Inc., (Warrington, PA).

In an embodiment, the concentration agent particles comprise particles of metal carbonates. A specific example of concentration agent metal carbonate particles is calcium carbonate, such as calcium carbonate particles having a diameter range of 2.5-10 microns that are commercially available from Sigma-Aldrich, (St. Louis, MO).

In an embodiment, the concentration agent particles comprise particles of metal phosphates. A specific example of concentration agent metal phosphate particles is hydroxyapatite, such type-1 hydroxyapatite particles having particle sizes from 2-8 microns that are commercially available from Sigma-Aldrich, (St. Louis, MO) and BioRad (Hercules, CA).

In one specific method, the nonwoven article is prepared using a wet laying or "wetlaid" process. In this process, a dispersion is formed that contains (a) a plurality of fibers, (b) a plurality of concentration agent particles, (c) polymeric binder fibers (e.g., bi-component fibers), (d) and a dispersing liquid such as water, a water-miscible organic solvent, or a mixture thereof. The fibers and concentration agent particles can be dispersed together in the dispersing liquid. In some embodiments, the fibers (for example, hydrophobic fibers) have additives, surface treatments, or chemical groups that facilitate dispersion of the fibers in the dispersion liquid. For example, polyolefin-based fibers can have maleic anhydride or succinic anhydride functionality, or, during the melt-processing to prepare polyolefin-based fibers, a suitable surfactant can be added.

The wetlaid process additionally includes dewatering, followed by heating to finish the dewatering and optionally to bind some of the fibers together.

One or more adjuvants or additives are optionally used in preparing this type of nonwoven article. Useful adjuvants include process aids, surfactants, solvents, dispersants, flocculating aids, retention aids, or other materials that can enhance the overall performance of the resulting nonwoven article. When used, the amounts of such adjuvants can be present, for example, in an amount up 5 weight percent, up to 4 weight percent, up to 3 weight percent, up to 1 weight percent, or up to 0.5 weight percent based on a total dry weight of the nonwoven article (for example, fibers and concentration agent particles). The total amount of adjuvants is typically selected to be as low as possible so as to maximize the amount of concentration agent particles that can be included in the nonwoven article.

In one more specific wetlaid process, the fibers (for example, chopped fibers) can be blended in a container in the presence of the dispersing liquid (for example, water, a water-miscible organic solvent such as an alcohol, or a mixture thereof) to form a slurry. After formation of the slurry, the concentration agent particles and an optional precipitation agent (for example, a pH adjusting agent such as alum) can be added to the slurry.

When the wetlaid process is carried out by using hand-sheet methods known in the art, the order of addition of the components (i.e., fibers and concentration agent particles) to the dispersion has not been found to significantly affect the ultimate performance of the concentration device. After formation, the dispersion mixture can be poured into a mold, the bottom of which can be covered by a screen. The dispersing liquid can be allowed to drain from the mixture (in the form of a wet sheet) through the screen. After sufficient liquid has drained, the wet sheet generally can be removed from the mold and dried by pressing, heating, or a combination of the two. Generally pressures are in a range of about 300 to about 600 kPa. Temperatures in a range of 90°C to 200°C, in a range of 100°C to 175°C, in a range of 100°C to 150°C, or in a range of 90°C to 120°C can be used for drying the wet sheet. Drying often removes all or most of the dispersing liquid (for example, up to 85 weight percent, up to 90 weight percent, up to 95 weight percent, up to 98 weight percent, or up to 99 weight percent of the dispersing liquid based on the amount of dispersing liquid added to form the dispersion).

The resulting nonwoven article is a dry sheet having an average thickness of at least 0.1 millimeter, at least 0.2 millimeters, at least 0.5 millimeters, at least 0.8 millimeters, at least 1 millimeter, at least 2 millimeters, at least 4 millimeters, or at least 5 millimeters. The average thickness is often up to 20 millimeters, up to 15 millimeters, up to 12 millimeters, or up to 10 millimeters. Calendering can be used to provide additional pressing or fusing, if desired, of the dry sheet. The basis weight of the nonwoven article (in the form of sheet material) can be in the range of about 100 to about 350 grams per square meter (g/$m^2$), preferably, in the range of about 200 to about 300 g/$m^2$, such as about 250 g/$m^2$.

In the nonwoven article, the concentration agent particles can be entrapped in the fibrous porous matrix through either chemical interactions (for example, chemical bonding) or physical interactions (for example, adsorption or mechanical entrapment), depending upon the nature of the fibers that are utilized. The concentration agent particles are often preferably distributed essentially uniformly throughout the fibrous porous matrix within the nonwoven article.

Generally the average pore size of the dry nonwoven article can be in a range of 0.1 to 10 micrometers, as measured by scanning electron microscopy (SEM). Void volumes in the range of 20 to 80 volume percent or in a range of 40 to 60 volume percent can be useful. The porosity of the dry nonwoven article can be modified (increased) by using fibers of larger diameter or stiffness in the fiber mixture.

Advantageously, nonwoven articles according to at least certain aspects of the present disclosure can be subjected to sterilization processes with minimal to no damage to the nonwoven articles. Suitable sterilization methods are known to the skilled practitioner, and include for instance and without limitation, steam treatment at a temperature of 121 degrees Celsius for at least 15 minutes, exposure to ethylene oxide, and gamma irradiation of the nonwoven articles.

A variety of microorganisms can be concentrated and detected by using the nonwoven articles and methods of the disclosure, including, for example, bacteria, fungi, yeasts, protozoans, viruses (including both non-enveloped and enveloped viruses), fungal spores, bacterial endospores (for example, *Bacillus* (including *Bacillus anthracis, Bacillus cereus,* and *Bacillus subtilis)* and *Clostridium* (including *Clostridium botulinum, Clostridium difficile,* and *Clostridium perfringens)),* and the like, and combinations thereof, such as gram-negative bacteria, gram-positive bacteria, yeasts, fungi, and combinations thereof. Target cellular analyte that can be concentrated and detected by using the methods of the disclosure include nucleic acids, proteins, adenosine triphosphate (ATP), or combinations thereof.

Capturing or binding of microorganisms, cellular analytes, or combinations thereof is accomplished by contacting a fluid sample with a nonwoven article. In certain embodiments, contacting comprises filtration of the fluid sample through the nonwoven article. In select embodiments, contacting comprises passing the fluid sample at least twice through the nonwoven article, such as by sending a filtrate through the nonwoven article a second time or by placing the nonwoven article into a volume of the fluid sample and agitating the fluid sample such that sample passes through the nonwoven article multiple times. The method optionally further comprises passing the fluid sample through a coarse filter prior to the contacting the fluid sample with the nonwoven article. The use of such a filter can remove particulates from the fluid sample that might otherwise clog the nonwoven article. Suitable coarse filters include for example and without limitation, filters comprising pore sizes of at least 1 micrometer, at least 5 micrometers, at least 10 micrometers, at least 25 micrometers, or at least 50 micrometers.

Advantageously, the nonwoven articles of the present disclosure require only a very low pressure differential across the nonwoven article to effectively pass a fluid sample through the nonwoven article. This characteristic is particularly beneficial in environments, for instance, in a field situation, and/or when no or low power pumps are available for transporting a fluid sample. In an embodiment of the present disclosure, the contacting comprises passing the fluid sample through the nonwoven article at a pressure of 14.7 pounds per square inch (psi) (101.3 kilopascals (kPa)) or less, or 4.0 pounds per square inch (psi) (27.58 kilopascals (kPa)) or less, or 3.0 psi (20.68 kPa), or 2.0 psi (13.79 kPa), or 1.0 psi (6.9 kPa), or 0.9 psi (6.21 kPa), or 0.8 psi (5.52 kPa), or 0.7 psi (4.83 kPa), or 0.6 psi (4.14 kPa), or even 0.5 psi (3.45 kPa)or less, and at a pressure of at least 0.4 psi (2.76 kPa), or at least 0.5 psi (3.45 kPa).

In certain embodiments, the method further comprises washing the at least one microorganism strain- or target cellular analyte-bound nonwoven article prior to placing the at least one microorganism strain- or cellular analyte-bound nonwoven article in contact with at least one detection reagent. It has been discovered that contaminants such as residual sample matrix can be removed from the nonwoven article without significant loss of the bound or captured microorganisms and/or cellular analytes. In certain embodiments, washing includes for instance and without limitation, rinsing with sterile deionized water or bottled drinking water, or rinsing with aqueous salt or buffer solutions. Washing the nonwoven article tends to remove components that could otherwise interfere with detecting the presence of the bound microorganisms and/or cellular analytes, depending on the particular detection method employed.

In certain embodiments, placing the microorganism strain- or target cellular analyte-bound nonwoven article in contact with a reagent includes placing the nonwoven article in a receptacle that comprises a material through which a detection signal can be detected, wherein the receptacle contains at least one reagent. For instance, placing the microorganism strain- or target cellular analyte-bound nonwoven article in contact with a reagent optionally includes placing the nonwoven article in a receptacle configured to be operationally connected to a luminometer, wherein the receptacle contains at least one reagent. Hence, in such an embodiment, detection is facilitated by disposing the receptacle in the luminometer for measurement of light generated from reaction of the bound microorganism strain and/or target cellular analyte with at least one reagent. Similarly, the receptacle can be interfaced with other types of equipment depending on the particular detection method. In certain embodiments, placing the microorganism strain- or target cellular analyte-bound nonwoven article in contact with a reagent includes pushing the nonwoven article into a receptacle containing the at least one reagent. It has been discovered that microorganism strain and/or target cellular analyte can be detected without requiring removal from being captured by the nonwoven article. The ability to detect microorganism strains and/or target cellular analytes attached to the nonwoven article is advantageous because it decreases the number of required method steps as compared to methods in which the microorganism strains and/or target cellular analytes need to be eluted from a nonwoven article prior to detection. Further, the nonwoven article concentrates the microorganism strains and/or target cellular analytes into the volume of the article, which is typically significantly smaller than the volume of the fluid sample contacted with the nonwoven article. Suitable devices and/or methods for use with the nonwoven articles of the present disclosure are disclosed in US 2018/051313.

Microorganisms and/or cellular analytes that have been captured or bound (for example, by adsorption, absorption, or by sieving) by the nonwoven article can be detected by essentially any desired method that is currently known or hereafter developed. Such methods include, for example, culture-based methods, microscopy (for example, using a transmitted light microscope or an epifluorescence microscope, which can be used for visualizing microorganisms tagged with fluorescent dyes) and other imaging methods, immunological detection methods, and genetic detection methods. The detection process following microorganism and/or cellular analyte capture optionally can include washing to remove sample matrix components, staining, boiling or using elution buffers or lysis agents to release cellular analyte

from the concentration device, or the like.

Immunological detection is detection of an antigenic material derived from a target organism, which is commonly a biological molecule (for example, a protein or proteoglycan) acting as a marker on the surface of bacteria or viral particles. Detection of the antigenic material typically can be by an antibody, a polypeptide selected from a process such as phage display, or an aptamer from a screening process.

Immunological detection methods are well-known and include, for example, immunoprecipitation and enzyme-linked immunosorbent assay (ELISA). Antibody binding can be detected in a variety of ways (for example, by labeling either a primary or a secondary antibody with a fluorescent dye, with a quantum dot, or with an enzyme that can produce chemiluminescence or a colored substrate, and using either a plate reader or a lateral flow device).

Detection can also be carried out by genetic assay (for example, by nucleic acid hybridization or primer directed amplification). The captured or bound microorganisms can be lysed to render their genetic material (e.g., cellular analytes) available for assay. Lysis methods are well-known and include, for example, treatments such as sonication, osmotic shock, high temperature treatment (for example, from about 50°C to about 100°C), and incubation with an enzyme such as lysozyme, glucolase, zymolose, lyticase, proteinase K, proteinase E, and viral enolysins.

Many commonly-used genetic detection assays detect the nucleic acids of a specific microorganism, including the DNA and/or RNA. The stringency of conditions used in a genetic detection method correlates with the level of variation in nucleic acid sequence that is detected. Highly stringent conditions of salt concentration and temperature can limit the detection to the exact nucleic acid sequence of the target. Thus microorganism strains with small variations in a target nucleic acid sequence can be distinguished using a highly stringent genetic assay. Genetic detection can be based on nucleic acid hybridization where a single-stranded nucleic acid probe is hybridized to the denatured nucleic acids of the microorganism such that a double-stranded nucleic acid is produced, including the probe strand. One skilled in the art will be familiar with probe labels, such as radioactive, fluorescent, and chemiluminescent labels, for detecting the hybrid following gel electrophoresis, capillary electrophoresis, or other separation method.

Particularly useful genetic detection methods are based on primer directed nucleic acid amplification. Primer directed nucleic acid amplification methods include, for example, thermal cycling methods (for example, polymerase chain reaction (PCR), reverse transcriptase polymerase chain reaction (RT-PCR), and ligase chain reaction (LCR)), as well as isothermal methods and strand displacement amplification (SDA) (and combinations thereof; preferably, PCR or RT-PCR). Methods for detection of the amplified product are not limited and include, for example, gel electrophoresis separation and ethidium bromide staining, as well as detection of an incorporated fluorescent label or radio label in the product. Methods that do not require a separation step prior to detection of the amplified product can also be used (for example, real-time PCR or homogeneous detection).

Bioluminescence detection methods are well-known and include, for example, adensosine triphosphate (ATP) detection methods including those described in U.S. Patent No. 7,422,868 (Fan et al.). Bioluminescence detection methods for ATP often include the known luciferin-luciferase system in which luciferase enzyme catalyzes the oxidation of luciferin in the presence of ATP and a divalent cation (such as magnesium or calcium). Other luminescence-based detection methods can also be utilized.

In many embodiments, detection comprises a culture-based detection method, an imaging detection method, a fluorescence-based detection method, a colorimetric detection method, an immunological detection method, a genetic detection method, a bioluminescence-based detection method, or a combination thereof.

In certain embodiments, contacting comprises passing the fluid sample through the nonwoven article using a sample delivery system. One suitable sample delivery system comprises a freestanding container comprising a first reservoir and a deformable self-supporting receptacle dimensioned to be received in the first reservoir of the freestanding container and comprising a second reservoir. The freestanding container is more rigid than the deformable self-supporting receptacle, and the freestanding container includes a base comprising an aperture formed therein, through which the deformable self-supporting receptacle can be accessed. Accordingly, fluid sample is passed through the nonwoven article by applying external pressure to the deformable self-supporting receptacle via the aperture in the freestanding container to pass the fluid sample through the nonwoven article.

In a second aspect, a method of detecting microorganisms and/or cellular analytes in a fluid sample is provided. The method includes a) providing a nonwoven article according to the first aspect and b) providing a fluid sample suspected of containing at least one microorganism strain or target cellular analyte. The method further includes c) contacting the fluid sample with the nonwoven article such that at least a portion of the at least one microorganism strain or target cellular analyte is bound to the nonwoven article and d) detecting the presence of the at least one bound microorganism strain or bound target cellular analyte.

In certain embodiments, suitable devices for contacting a fluid sample with a nonwoven article are as described in US 2018/051313. The device includes a sample container, a filter holder, a nonwoven article, and a first adaptor configured to interface the filter holder with a receptacle. The nonwoven article includes a fibrous porous matrix and a plurality of concentration agent particles enmeshed in the fibrous porous matrix. In many embodiments, the first adaptor comprises a hollow shape (e.g., a hollow cylinder) having a ledge on which the nonwoven article is placed.

Nonlimiting examples of suitable receptacles, for instance receptacles containing at least one reagent, include the 3M CLEAN-TRACE Surface ATP Swab available from 3M Company (St. Paul, Minn.), the AQUASNAP ATP Water Test available from Hygiena (Camarillo, Calif.), the ACCUPOINT 2 ATP Sanitation Monitoring System available from Neogen Corporation (Lansing, Mich.), and the PRO-CLEAN Rapid Protein Residue Test available from Hygiena.

Advantageously, a relatively large volume of fluid sample is optionally passed through the nonwoven article to concentrate the target microorganism and/or target cellular analyte the container comprises a volume of at least 50 milliliters, or at least 100 milliliters, or at least 150 milliliters, or at least 300 milliliters, or at least 500 milliliters, or up to 1 liter.

Following contact of a fluid sample with the nonwoven article, the nonwoven article is readily placed in contact with at least one detection reagent. In certain embodiments, the nonwoven article can be moved using forceps, while in other embodiments the nonwoven article can be pushed into a receptacle and thereby into contact with one or more detection reagents contained in the receptacle.

Endoscopy procedures are performed using complex, reusable, flexible instruments that, when inserted into the body, may become heavily contaminated with patient biomaterial and microorganisms, including potential pathogens. Careful reprocessing of flexible endoscopes between patients is critical to reducing the risk of cross-contamination and the possible transmission of pathogens. Cleaning for these devices is typically defined as the removal of visible soil (e.g., organic and inorganic material) from objects and surfaces and it is accomplished manually or mechanically using water with detergents or enzymatic products. Failure to perform proper cleaning leaves behind organic and inorganic residues that may interfere with the disinfection process increasing the risk for reprocessing failures and patient infection. Thus, the need to evaluate the efficacy of cleaning and disinfection has been recognized as an important part of flexible endoscope reprocessing. A visually based method of verification, however, has severe limitations when applied to flexible endoscopes because the complex, narrow lumens in these devices cannot be directly visually inspected. Use of methods according to the present disclosure, which may be performed in real time, to test a rinsate from instruments following cleaning provides an opportunity to take any corrective action required such as re-cleaning and reprocessing.

In certain embodiments of the method of the disclosure, the fluid sample comprises a rinsate from a lumened or cannulated device and the contacting occurs in an integrated assembly in which the lumened or cannulated device is disposed in fluid communication with the nonwoven article. For instance, the integrated assembly optionally comprises a tube that places the lumened or cannulated device in fluid communication with the nonwoven article. In an embodiment, in the integrated assembly, the lumened or cannulated device is connected to the nonwoven article. In such embodiments, the rinsate may flow from the device through the tube to the nonwoven article. Suitable lumened or cannulated devices include for example and without limitation, a flexible endoscope, a semi-rigid endoscope, a rigid endoscope, a laparoscopic instrument, or a cannulated robotic surgical instrument.

## EXAMPLES

Unless otherwise noted, all chemicals used in the examples can be obtained from Sigma-Aldrich Corp. (Saint Louis, MO). Unless otherwise specified, all microbiological supplies and reagents were purchased as standard products from either Sigma-Aldrich or VWR.

| Material | Vendor |
|---|---|
| Fiber 1 - SHORT STUFF E380F ~0.7 mm average length, 15 microns diameter polyethylene fibers | MiniFIBERS, Inc.; Johnson City, TN |
| Fiber 2 - 6 denier 2 inches long chopped nylon fibers | MiniFIBERS, Inc.; Johnson City, TN |
| Fiber 3 - 1 denier bi-component ethylene vinyl acetate/polypropylene fibers | MiniFIBERS, Inc.; Johnson City, TN |
| Fiber 4 - long glass fibers (MICRO-STRAND 106-475 Glass Fiberglas) Schuller Inc. | Johns Mansville; Denver, CO |
| Fiber 5 - 0.06 denier, 2.5 microns diameter, 1.5 mm in length polyester/copolyester fibers | Cyphrex 10001 fibers from Eastman Co., Kingsport, TN |
| Fiber 6 - 2 denier 5 mm bi-component copolyester fibers made of polyester as the core and PET as the sheath | MiniFIBERS, Inc.; Johnson City, TN |
| Fiber 7 - typical length 4.3 mm (range 4.5-7.5 mms), specific gravity 1.17 acrylonitrile fibers | CFF Fibrillated Fiber 114-3 from Sterling Fibers, Inc. Pace, FL |
| CM-111 - Amorphous spheroidized magnesium silicate: Cosmetic Microspheres (CM-111) | 3M Company; St. Paul, MN |

(continued)

| Material | Vendor |
|---|---|
| G-CM-111 Guanylated CM-111 made according to the method of Example E1-D in PCT/US2014/040861 | 3M Company; St. Paul, MN |
| G-Perlite Guanylated Perlite made according to copending US Serial No. 62/135,303 (Docket 76251US002) | 3M Company; St. Paul, MN |
| Hydroxyapatite - Product # 289396, 20 micrometer average diameter | Sigma Aldrich; St. Louis, MO |
| Hydroxyapatite - Type II, grade CHT, 20 micrometer average diameter | BioRad; Hercules, CA |
| DI Water - Deionized filtered 18 megaohm water from a Milli-Q Gradient System | Millipore; Waltham, MA |
| ATP free water - HYPURE Molecular biology grade water, Catalog # SH30538.02 | Thermo Fisher Scientific; Waltham, MA |
| CLEAN-TRACE lysis reagent - reagent for bioluminescence assay | 3M Company; Bridgend, UK |
| CLEAN-TRACE luciferin-luciferase enzyme reagent - reagent for bioluminescence assay | 3M Company; Bridgend, UK |
| Tryptic Soy Broth - DIFCO Tryptic Soy Broth, prepared at 3% according to the manufacturer's instructions | Becton Dickenson; Sparks MD |
| *E. coli* plate - 3M *E coli*/Coliform PETRIFILM Plate; | 3M Company; St. Paul MN |
| Endo Agar plate - Premade agar plate, Catalog # 254016 | Becton Dickenson; Sparks MD |
| TSA plate - plates prepared according to manufacturer's instructions with 3 wt% Tryptic Soy Agar powder | Becton Dickenson; Sparks MD |
| YPD Agar plate - plate prepared according to manufacturer's instructions with 5 wt% Yeast Extract Peptone Dextrose and 1.5 wt% agar | Becton Dickenson; Sparks MD |
| PAC - 3M PETRIFILM Aerobic Count Plates | 3M Company; St. Paul MN |
| BBL Buffer - Butterfield's buffer, pH 7.2 ± 0.2, monobasic potassium phosphate buffer solution (VWR Catalog Number 83008-093) | VWR; West Chester, PA |
| 3M CLEAN-TRACE NG luminometer | 3M Company, Bridgend, UK |
| Cuvettes - Greiner Bio-One polystyrene 4 mL tubes | VWR; West Chester, PA |
| Microfuge tubes - 1.5 mL BrandTech polypropylene tubes | VWR; West Chester, PA |

*Preparation of nonwoven fibrous porous matrices containing calcined magnesium silicate*

**Examples 1, 2 and 3:** Three fiber premixes were prepared by mixing various amounts of Fiber 1, Fiber 2, Fiber 3, and Fiber 4 as shown in Table 1 below. The fibers were added to 3 liters of cold deionized water in a 4 L blender (available from VWR, Radnor, PA, under the trade designation "WARING COMMERCIAL HEAVY DUTY BLENDER, MODEL 37BL84") and blended at low speed for 30 seconds. The mixture was examined for uniform dispersion of the fibers without nits or clumps. The additive particles, CM-111, were added with an additional liter of deionized water and mixed at low speed for 15 seconds.

A nonwoven fibrous porous felt was prepared using a pad maker apparatus (obtained from Williams Apparatus, Watertown, NY, under the trade designation "TAPPI") that had a box measuring about 30 centimeters (12 inches) square and 30 centimeters (~ 12 inches) high with a fine mesh screen at the bottom and a drain valve. On the screen ~ a 14 inch (36 cm) x 12 inch (30 cm) piece of a polyethylene spunbond (PET Lutradur 7240 obtained from Fiberweb, Cincinnati, OH) was laid as scrim on the screen. The box was filled with tap water up to a height of about 1 centimeter above the screen. Each fiber and particle mixture was poured into the box and the valve was opened immediately which created a vacuum that pulled the water out of the box.

The fibrous nonwoven felts were each transferred from the apparatus onto a 20 centimeter square sheet of blotter paper (96-pound white paper, obtained from Anchor Paper, St. Paul, MN). Each felt was sandwiched between 2 to 4 layers of blotter paper, to blot excess water. The pressed felt was then transferred onto a fresh sheet of blotter paper

and placed in an oven (obtained from SPX Thermal Product Solutions, White Deer, PA, under the trade designation "BLUE M STABIL-THERM OVEN, MODEL OV-560A2") set at 110°C for about 3 hours to remove residual water and to form a nonwoven fibrous porous matrix. The resulting fibrous porous matrices of Examples 1 and 2 were approximately 0.8-1 millimeter thick. The fibrous porous matrix of Example 3 was approximately 0.8-0.9 millimeter thick.

FIGS. 1-3 are scanning electron microscope (SEM) images of the exemplary nonwoven articles of Examples 1-3, respectively.

**Table 1: Compositions of Examples 1-3 wherein examples 1 and 3 are comparative examples**

| Materials (in grams) | Example 1 | Example 2 | Example 3 |
|---|---|---|---|
| Fiber 1 | 11.08 | 10.98 | 8.83 |
| Fiber 2 | 3.01 | 0 | 2.44 |
| Fiber 3 | 2.30 | 2.29 | 1.82 |
| Fiber 4 | 0 | 1.8 | 1.4 |
| CM-111 | 5.15 | 5.07 | 5.03 |
| Basis weight (g/m$^2$) | 196.66 | 203.44 | 197.74 |

*Preparation of nonwoven fibrous porous matrices containing guanylated magnesium silicate*

**Examples 4, 5, 6, 7 and 8 wherein examples 6-8 are comparative examples: Nonwoven fibrous** porous matrices of Examples 4, 5, 6, 7, and 8 were made using the procedure described above. The formulations are shown in Table 2 below.

**Table 2: Compositions of Examples 4-8**

| Materials (in grams) | Example 4 | Example 5 | Example 6 | Example 7 | Example 8 |
|---|---|---|---|---|---|
| Fiber 1 | 11.03 | 11.05 | 11.08 | 11.05 | 11.01 |
| Fiber 2 | 0 | 0 | 3.02 | 3.03 | 3.01 |
| Fiber 3 | 2.25 | 2.26 | 2.27 | 2.25 | 2.26 |
| Fiber 4 | 2.25 | 0 | 1.76 | 0 | 1.76 |
| Guanylated CM-111 | 5.00 | 5.00 | 5.01 | 5.00 | 5.00 |
| Basis weight (g/m$^2$) | 217.07 | 166.30 | 238.32 | 199.02 | 229.82 |

The thickness of the fibrous porous matrices of Example 4 was approximately 0.8-0.9 mm, Example 5 was approximately 0.6-0.8 mm thick, and Examples 6-8 were each approximately 0.8-1.0 mm thick.

**Example 9:** Bacteria used in examples

The various bacteria used in the examples (see Table 3 below) were obtained from ATCC (Manassas, VA).

**Table 3. Bacteria used in examples**

| Bacteria | ATCC No. |
|---|---|
| *Escherichia coli* | 51813 |
| *Staphylococcus aureus* | 6538 |
| *Enterobacter aerogenes* | 29007 |
| *Pseudomonas aeruginosa* | 9027 |

Pure cultures of the bacterial strains were inoculated into Tryptic Soy Broth (TSB, Becton, Dickinson and Company, Franklin Lakes, NJ) and were grown overnight at 37°C. The cultures were diluted serially in Butterfield phosphate buffer (3M Co., St. Paul, MN) to obtain desired amount of colony forming units (cfu) per ml for spiking into water

samples. *E. coli* and *E. aerogenes* were quantified by plating appropriate dilutions on 3M PETRIFILM *E. coli*/Coliform Count Plates (3M Company) according to manufacturer's instructions and incubated overnight at 37°C. *S. aureus* and *P. aeruginosa* were quantified by plating appropriate dilutions on 3M PETRIFILM aerobic count plates (3M Company) and incubated overnight at 37°C. The plates were read using 3M PETRIFILM Plate Reader (3M Co.) and colony forming units (cfu) were determined.

**Example 10:** Bacterial capture efficiency of nonwoven fibrous porous matrices

100 ml of either neutralized tap water or 18 mega ohms double deionized water was spiked with various bacteria to get 100 cfu/ml (total of 10,000 cfu in 100 ml). A 14 mm disk was cut from the various nonwoven fibrous porous matrices described above and the disk was placed in a SWINNEX 13 Filter Holder (Catalog number SX0001300, EMD Millipore, Billerica, MA). The filter holder was closed and attached to a 60 mL BD syringe with BD LUER-LOK tip (Product Number 309653, Becton, Dickinson and Company). The plunger from the syringe was removed prior to attaching the filter holder. The syringe was connected to a 12-place Waters Millipore SEP-PAK vacuum manifold (Waters Corporation, Milford, MA). A collection tube was placed in the sample rack to collect the filtrate from each of the syringes. The vacuum manifold was attached to Air Cadet Vacuum/Pressure Station (model No. 420-3901, Barnant Company, Barrington, IL) and the solution was filtered through the fibrous porous matrix materials at a vacuum pressure of about 15 inches of mercury. The filtration took less than a minute. For manual filtration, the samples were also filtered by pushing the syringe plunger through the barrel.

The filtrate from each of the filtrations was collected in sterile tubes and 1 ml of each was plated on PETRIFILM plates to enumerate the amount of bacteria left over in the solution. The plates were incubated overnight at 37°C and colonies growing on the plates were enumerated. The input sample was also plated prior to filtration to determine the amount of bacteria in the sample. The samples were plated on a minimum of three plates and experiments were repeated several times. The number of bacteria in the input solution and the number of bacteria left over in the filtrate was used to calculate the percent capture efficiency, according to the following formula:

$$\% \text{ Capture Efficiency} = (\text{RLUs from test nonwoven fibrous porous disk/RLUs from the 100\% Control}) \times 100$$

The bacterial capture ranged from 32 to 95 percent depending on the material (see Tables 4, 5, 6 and 7 below). The nonwoven fibrous porous matrices without fiberglass (Fiber 4) ranged from 32 to 70%. The percent capture of the nonwoven fibrous porous matrices without nylon ranged from 90 to 95%. Similar results were seen with *E. aerogenes* and *P. aeruginosa,* as also shown in Tables 4 and 6 below.

The percent capture of bacteria in nonwoven fibrous porous matrices without Fiber 2 (nylon) indicated that it was not essential for bacterial capture. However, Fiber 4 (fiberglass) was more important as the percent capture dropped to 32 to 70% when Fiber 4 was removed. This was the case with nonwoven fibrous porous matrices containing either type of particles (calcined magnesium silicate or guanylated magnesium silicate).

**Example 11:** Detection of bacteria in nonwoven fibrous porous matrices by ATP bioluminescence.

Each nonwoven fibrous porous matrix containing bound bacteria was removed from the SWINNEX filter holder and transferred aseptically to a 1.5 ml microfuge tube (Plastibrand microtubes, Brand GmbH & Co. KG, Wertheim, Germany). 200 microliters of CLEAN-TRACE lysis reagent) containing bacterial lysis reagent was added to the tube and vortexed for 1 minute and allowed to sit at room temperature for additional 1 minute. 250 microliters of CLEAN-TRACE luciferin-luciferase enzyme reagent) was added to the tube and mixed. The tube was placed immediately into a bench-top luminometer (20/20n single tube luminometer, Turner Biosystems, Sunnyvale, CA) and measurement of RLUs was recorded. The luminescence measurements were obtained from the luminometer using spreadsheet interface PC software that was provided with the luminometer. 100 microliters of buffer containing 10,000 cfu of bacteria was pipetted into a 1.5 ml microfuge tube (Plastibrand microtubes) and upon addition of lysis mix (200 microliters) and ATP reagent (250 microliters), luminescence was measured. The relative light units obtained from 10,000 cfu was used to calculate the percent recovery of ATP in nonwoven fibrous porous matrices (see Tables 4 and 5 below). The ATP recovery varied from 30 to 68% with uncut material and the matrix without Fiber 2 gave the most recovery (60 to 68%).

**Table 4. Bacterial capture efficiency and percent recovery of ATP in uncut nonwoven fibrous porous matrices containing calcined magnesium silicate**

| Matrix | EC 10,000 cfu total | | SA 10,000 cfu total | |
|---|---|---|---|---|
| | % Capture | % ATP Recovery | % Capture | % ATP Recovery |
| Example 1 | 60 | 32 | 32 | 30 |
| Example 2 | 94 | 62 | 92 | 60 |
| Example 3 | 77 | 48 | 85 | 45 |
| | EA 10,000 cfu total | | PA 10,000 cfu total | |
| Matrix | % Capture | % ATP Recovery | % Capture | %ATP Recovery |
| Example 1 | 48 | 35 | 38 | 30 |
| Example 2 | 95 | 55 | 90 | 58 |
| Example 3 | 65 | 35 | 62 | 30 |

**Table 5. Bacterial capture efficiency and percent recovery of ATP in uncut nonwoven fibrous porous matrices containing guanylated magnesium silicate**

| Matrix | EC 10,000 cfu total | | SA 10,000 cfu total | |
|---|---|---|---|---|
| | % Capture | % ATP Recovery | % Capture | % ATP Recovery |
| Example 4 | 60 | 56 | 70 | 55 |
| Example 5 | 93 | 65 | 95 | 68 |
| Example 6 | 85 | 46 | 91 | 49 |

In another experiment, the nonwoven fibrous porous matrix was aseptically cut into small pieces and then transferred to 1.5 ml microfuge tube (Plastibrand microtubes). 200 microliters of the lysis mix containing bacterial lysis reagent was added to the tube and vortexed for 1 minute and allowed to sit at room temperature for an additional 1 minute. 250 microliters of the ATP reagent containing luciferin and luciferase was added to the tube and mixed. The luminescence was measured as described above. The percent recovery of ATP in nonwoven fibrous porous matrices is shown in Tables 6 and 7 below. The ATP recovery varied from 55 to 77% with uncut material and the wet-laid without Fiber 2 gave the most recovery (70 to 77%).

**Table 6. Bacterial capture efficiency and percent recovery of ATP in cut fibrous porous matrices containing calcined magnesium silicate**

| Matrix | EC 10,000 cfu total | | SA 10,000 cfu total | |
|---|---|---|---|---|
| | % Capture | % ATP Recovery | % Capture | % ATP Recovery |
| Example 1 | 55 | 60 | 45 | 55 |
| Example 2 | 92 | 77 | 90 | 70 |
| Example 3 | 75 | 65 | 82 | 72 |
| | EA 10,000 cfu total | | PA 10,000 cfu total | |
| Matrix | % Capture | % ATP Recovery | % Capture | % ATP Recovery |
| Example 1 | 45 | 55 | 35 | 40 |
| Example 2 | 90 | 68 | 90 | 65 |
| Example 3 | 68 | 52 | 65 | 55 |

**Table 7. Bacterial capture efficiency and percent recovery of ATP in cut fibrous porous matrices containing guanylate magnesium silicate**

| Matrix | EC_10,000 cfu total | | SA_10,000 cfu total | |
|---|---|---|---|---|
| | % Capture | % ATP Recovery | % Capture | % ATP Recovery |
| Example 4 | 62 | 55 | 68 | 57 |
| Example 5 | 91 | 68 | 90 | 70 |
| Example 6 | 83 | 52 | 88 | 45 |

**Example 13:** Testing of fibrous porous matrices with various formulations for removal *of E. coli* from water samples by filtration

A streaked culture of *E coli* (ATCC 11229) on a TSA was incubated overnight at 37°C. From the plate an isolated colony was removed and inoculated into 5 ml of TSB using a standard microbiology loop and incubated in a shaking incubator (INNOVA 44 from New Brunswick Scientific) at 37°C for 20-22 hours. The overnight culture that contained ~ 2-3 $\times$ $10^9$ cfu/ml was serially diluted in Butterfield's Buffer to obtain an inoculum with approximately 1 $\times$ $10^6$ cfu/ml.

A test sample was prepared by inoculating 200 ml deionized of water (MilliQ Gradient system, Millipore, Ma) a 1:100 dilution of the $10^6$ cfu/ml inoculum resulting in water test sample containing approximately $10^4$ cfu/ml (~ 4 Log cfus/ml).

A disk 47 mm in diameter was die punched from the nonwoven fibrous porous matrix of Example 1 and placed into a sample holder which was a custom device fabricated from polycarbonate. The device had three parts and was cylindrically shaped measuring about 60 cm in diameter by about 45 cm high. The lower part contained a support screen for the filter disk and a sample outlet port. The top portion was enclosed except for the sample inlet port through PVC tubing connected to the Cole Parmer peristaltic pump and was vented on the upstream side to allow for purging air. O-ring seals were used to prevent leakage on both the upstream and downstream sides. Internal threads provided closure pressure. The 47 mm disk was placed on top of the support screen, an O ring was added on top, and the holder was closed.

The fibrous porous matrices were tested individually and without replicates. A pre-filtration sample was pumped through the sample holder containing the nonwoven matrix disk using a Cole Parmer peristaltic pump (Model No. 7553-70) using 1/8 inch (0.32 cm) wall thick PVC tubing (VWR catalog # 60985-522). The spiked water was pumped through the fibrous porous matrix at a flow rate of 12 ml/minute. Filtrates were collected in 250 ml sterile glass bottles. The first 100 ml filtrate was collected and discarded. The second 100 ml filtrate was collected for further processing. After each filtration test, the holder was disassembled to remove the fibrous porous matrix using sterile forceps. Between testing of the matrices the filtration device was rinsed with filtered sterilized 500 ml deionized water.

A 10 ml volume of the second 100 ml filtrate was added to a 100 ml containing Butterfield's Buffer flip-top bottle to obtain a 1:10 dilution. The bottle was capped and mixed manually by shaking for 10 seconds. A 10 ml volume was removed and added to another flip-top bottle to obtain a 1:100. Similarly the filtrate was further diluted to 1:1000 and 1:10000. These 100 ml diluted filtrates were vacuum filtered through 0.45 micron filters. After each filtration, the vacuum apparatus was rinsed with filtered sterilized 500 ml deionized water and blotted dry with Kimwipes.

The matrices were removed from the apparatus with sterile forceps plates and placed on Endo Agar plates, grid side up. The plates were incubated at 37°C for 18-20 hours. Colony counts were obtained from plates by manual counting. Pre-filtration samples were also diluted and filtered as the procedure above. The cfu/ml colony counts were converted to Log cfu/ml values. Log Reduction Values (LRV) were calculated based on counts obtained from the plated filtrate and pre-filtration samples by using the formula below.

$$LRV= (\text{Log of cfus/ml in pre-filtration sample}) - (\text{Log of cfus/ml in filtrate sample})$$

Filtration testing was done on 47 mm disks of the matrices of Examples 4, 5, 7, and 8. The results are shown in Table 8 below.

**Table 8. Log reduction value *of E. coli* using fibrous porous matrices containing calcined magnesium silicate**

| Matrix | Log cfus in pre-filtration sample | LRV |
|---|---|---|
| Example 4 | 4.53 | 3.53 |

(continued)

| Matrix | Log cfus in pre-filtration sample | LRV |
|---|---|---|
| Example 5 | 4.53 | 3.53 |
| Example 7 | 4.53 | 3.53 |
| Example 8 | 4.53 | 4.53 |

*Preparation of nonwoven fibrous porous matrices containing metal phosphate*

**Comparative Examples 14, 15, and Example 16**: Three fiber premixes were prepared by using various amounts of Fiber 1, Fiber 2, Fiber 3, Fiber 4, and hydroxyapatite (HA, from BioRad) as a metal phosphate concentration agent, as shown in Table 9 below. The nonwoven fibrous porous matrices were prepared according to the procedure described above for Examples 1-3. The resulting nonwoven fibrous porous matrix of Example 14 was approximately 0.8-1 millimeter thick. The nonwoven fibrous porous matrices of Examples 15 and 16 were 0.6-0.9 millimeters and 0.5-0.8 millimeters thick respectively.

**Table 9: Compositions of Examples 14-16**

| Material (in grams) | Comparative Example 14 | Comparative Example 15 | Example 16 |
|---|---|---|---|
| Fiber 1 | 11.03 | 11.02 | 11.10 |
| Fiber 2 | 3.00 | 3.04 | 0 |
| Fiber 3 | 2.24 | 2.26 | 2.28 |
| Fiber 4 | 1.76 | 0 | 1.83 |
| HA | 5.00 | 5.02 | 5.09 |
| Basis weight ($g/m^2$) | 242.61 | 196.85 | 209.94 |

**Example 17:** Testing of nonwoven fibrous porous matrix with hydroxyapatite for bacterial capture and ATP detection

A single colony from a streak culture *of E. coli* (ATCC 51813, a Gram negative organism) was inoculated into 10 ml of TSB (Tryptic Soy Broth, 3% by weight from Difco), and incubated overnight for about 20 hours at 37°C. The resulting bacterial stock contained about $1 \times 10^9$ cfus/ml. That stock was serially diluted in DI water to make a working stock of $1 \times 10^4$ cfus/ml.

14 mm disks of the nonwoven fibrous porous matrix of Example 14 were die punched and inserted into 13 mm filter holders (SWINNEX holders obtained from Millipore). One ml of the above working stock was filtered through the disk using a 1 cc syringe. The filtrate was discarded. The disk was removed from the holder and placed in a cuvette. A volume of 100 microliters of the CLEAN-TRACE lysis reagent was added to the disk and vortexed for 10 seconds. A volume of 300 microliters of CLEAN-TRACE luciferin-luciferase enzyme reagent was added to the cuvette and vortexed for 10 seconds. The cuvette was connected to the adaptor (custom made in 3M machine shop, 12 cm long, 1 cm in diameter made from DELRIN material (DuPont Co., Wilmington, DE)) and read in the NG luminometer. Disks though which 1 ml unspiked DI water was filtered were also tested for background ATP signal. Another set of disks was prepared the same as the background control by spiking the disks with a 100 microliter volume from a $1 \times 10^5$ cfus/ml dilution. This spiked disk was tested for ATP signal (in RLUs). This was the "100% control" sample. Capture efficiency was calculated using the formula of Example 10 above. 14 mm disks from the matrices of Examples 15 and 16 were tested as described for Example 14 above. The results for Examples 14-16 are shown in Table 10 below.

**Table 10: *E coli* bacterial capture efficiency of ATP in nonwoven fibrous porous matrices containing metal phosphate**

| Sample | Comparative Example 14 | Comparative Example 15 | Example 16 |
|---|---|---|---|
| ATP signal in RLUs in test disks | 572 | 747 | 612 |
| 100 % Control (ATP signal in RLUs | 855 | 1699 | 1081 |

(continued)

| Sample | Comparative Example 14 | Comparative Example 15 | Example 16 |
|---|---|---|---|
| % Capture Efficiency | 62 | 39 (45) | 53(20) |
| n=2, % std deviation indicated in parentheses if greater than 10%. Background ATP signals for the matrices of Examples 14, 15, and 16 were 36, 83 and 42 RLUS, respectively, and were each subtracted from the signals of the spiked disks. | | | |

Example 15 exhibits the most variability suggesting a critical role for fiberglass in detecting bacteria by bioluminescence. The nonwoven fibrous porous matrix of Example 15 may be more suitable for culture based or immunological detection embodiment than bioluminescence.

**Example 18:** Testing of nonwoven fibrous porous matrix with hydroxyapatite for rapid microbial monitoring in produced water samples

Produced water samples were obtained from an oil well in Canada. Samples were serially diluted in BBL and plated 1 ml each on PAC plates. The plates were incubated at 37°C for 48 hours per manufacturer instructions. The plates were analyzed for bacterial counts using the 3M PETRIFILM Plate Reader. A one hundred microliter volume from each of the samples was added to a cuvette and mixed with 145 microliters of the CLEAN-TRACE lysis reagent and vortexed for 10 seconds. A volume of 450 microliters of the CLEAN-TRACE luciferin-luciferase enzyme reagent was added, then mixed for 10 seconds. Using an adaptor (described above in Example 17) the cuvette was inserted into the NG luminometer to measure the ATP signal.

Based on the colony counts and ATP values two samples were further selected for testing with the matrices of Examples 1 to 3. Sample D (Comparative Example 1) had approximately $6.2 \times 10^4$ cfus/ml and an ATP signal of 1148 RLUs while sample G (Comparative Example 2) had $1.2 \times 10^5$ cfus/ml and an ATP signal of 243 RLUs.

14 mm disks of the nonwoven fibrous porous matrix of Example 14 were die punched and inserted into 13 mm filter holders (SWINNEX holders obtained from Millipore). Ten ml of produced water sample D (Comparative Example 1) was filtered through the disks using a 10 cc syringe. The filtrate was discarded. One disk was removed from the holder and placed in a cuvette and mixed with 145 microliters of the CLEAN-TRACE lysis reagent. The cuvette was vortexed for 10 seconds. A volume of 450 microliters of the CLEAN-TRACE luciferin-luciferase enzyme reagent was added, then mixed for 10 seconds. The cuvette was connected to the adaptor and read in the NG luminometer. This was Example 18a. A second disk was washed with 5 ml DI water and then analyzed for ATP signal the same as for the first disk. This was Example 8b. Disks through which 1 ml unspiked DI water was filtered were tested for background ATP signal. Background signal was less than 50 RLUs and was not subtracted from test readings. The improvement in ATP signal from captured bacteria over a CLEAN-TRACE test (without concentrating the bacteria in a nonwoven fibrous porous matrix) was calculated using the formula below. The same procedure was used for Examples 19a through 23b, using the nonwoven fibrous porous matrices of Examples 14-16 and either produced water sample D (Comparative Example 1) or produced water sample G (Comparative Example 2). Data is shown in Table 11 below.

$$\text{Fold increase in ATP signal over CLEAN-TRACE test} = (\text{RLUs from post filtration nonwoven matrix disk}/\text{RLUs from 100 microliters of unfiltered sample}).$$

**Table 11. ATP signal from produced water samples filtered through nonwoven porous matrices containing metal phosphate. Examples 18 a to 22b are comparative examples**

| Example # | Matrix | Disk for testing | Average ATP signal | Fold increase over CLEAN-TRACE test |
|---|---|---|---|---|
| Example 18a | Example 14 | Not washed | 5026.7 | 4 |
| Example 18b | Example 14 | Washed | 8290.4 | 7 |
| Example 19a | Example 15 | Not washed | 8928.7 | 8 |
| Example 19b | Example 15 | Washed | 19762.0 | 17 |
| Example 20a | Example 16 | Not washed | 7005.9 | 6 |

(continued)

| Example # | Matrix | Disk for testing | Average ATP signal | Fold increase over CLEAN-TRACE test |
|---|---|---|---|---|
| Example 20b | Example 16 | Washed | 8467.3 | 7 |
| Comparative Example 1 | N/A | N/A | 1148 | N/A |
| Example 21a | Example 14 | Not washed | 741.4 | 3 |
| Example 21b | Example 14 | Washed | 1127.8 | 5 |
| Example 22a | Example 15 | Not washed | 587.2 | 2 |
| Example 22b | Example 15 | Washed | 1030.0 | 4 |
| Example 23a | Example 16 | Not washed | 1084.6 | 4 |
| Example 23b | Example 16 | Washed | 1637.6 | 7 |
| Comparative Example 2 | N/A | N/A | 242 | N/A |
| N=2, stdev less than 10% unless otherwise noted | | | | |

The improvement in ATP signal after washing indicates the decrease in carryover of inhibitory substances, which allows the ATP assay to be used for rapid monitoring of industrial samples.

**Examples 24, 25, 26, 27, and 28:** Five fiber premixes were prepared by using various amounts of Fiber 1, Fiber 2, Fiber 3, Fiber 4, and hydroxyapatite (HA, from Sigma Aldrich) as a metal phosphate concentration agent, as shown in Table 12 below. The nonwoven fibrous porous matrices were prepared according to the procedure described above for Examples 1-3.

**Table 12: Compositions of Comparative Examples 24-27 and example 28**

| Material (in grams) | Example 24 | Example 25 | Example 26 | Example 27 | Example 28 |
|---|---|---|---|---|---|
| Fiber 1 | 11.00 | 11.05 | 11.02 | 11.07 | 11.01 |
| Fiber 2 | 3.02 | 3.01 | 3.01 | 3.08 | 0 |
| Fiber 3 | 2.25 | 2.25 | 2.25 | 2.32 | 2.26 |
| Fiber 4 | 1.76 | 1.75 | 1.75 | 0 | 1.79 |
| HA | 10.03 | 10.2 | 20.03 | 5.00 | 5.00 |
| Thickness (in mm) | 0.8-1.0 | 1.0-1.3 | 1.0-1.4 | 1.0-1.6 | 0.8-1.2 |

The nonwoven fibrous porous matrices of Examples 27 and 28 had compromised integrity such that the sheets included several holes, thus measurements were made in well-formed sections.

*Testing of nonwoven fibrous porous matrices with hydroxyapatite for rapid microbial monitoring in produced water samples*

Produced water samples were obtained from an oil well in Canada. Sample G was serially diluted in BBL and plated 1 ml each on PAC plates. The plates were incubated at 37°C for 48 hours per manufacturer instructions. The plates were analyzed for bacterial counts using the 3M PETRIFILM Plate Reader. A one hundred microliter volume from each sample was added to a cuvette and mixed with 145 microliters of the CLEAN-TRACE Water-Plus Total ATP extractant and vortexed for 10 seconds. A volume of 450 microliters of the CLEAN-TRACE Water-Plus Total ATP enzyme was added, then mixed for 10 seconds. Using an adaptor (described above in Example 14), the cuvette was inserted into a 3M CLEAN-TRACE NG luminometer to measure the ATP signal. Sample G (Comparative Example 3) contained $1.2 \times 10^5$ cfus/ml.

14 mm disks of the nonwoven fibrous porous matrix of Example 14 were die punched and inserted into 13 mm filter holders (SWINNEX holders obtained from Millipore). The disks were tested according to the procedure above for

Example 18. The nonwoven fibrous porous matrices were tested in duplicates. The average background signal of each matrix was subtracted from the test readings. The same procedure was used for Examples 30a through 34b, using the nonwoven fibrous porous matrices of Examples 24-28 and either produced water sample D (Comparative Example 3) or produced water sample G (Comparative Example 4). The improvement in ATP signal from captured bacteria over the Clean-Trace test (without concentration of bacteria by a nonwoven fibrous porous matrix) was calculated using the formula in Example 18 above. The data is shown in Table 13 below.

**Table 13: ATP signal from produced water samples filtered through nonwoven porous matrices containing metal phosphate: examples 29a to 33b are comparative examples**

| Example # | Matrix | Average Total ATP signal in RLUs | Fold increase over CLEAN-TRACE Total ATP |
|---|---|---|---|
| CE 3 | N/A | 964 | N/A |
| Example 29a not washed | 14 | 7279 | 7.55 |
| Example 29b washed | 14 | 3954 | 4.1 |
| Example 30a not washed | 24 | 15548 | 16.1 |
| Example 30b washed | 24 | 12690 | 13.1 |
| CE 4 | N/A | 1790 | N/A |
| Example 31a not washed | 25 | 36401(13) | 20 |
| Example 31b washed | 25 | 35235 | 19.6 |
| Example 32a not washed | 26 | 49454 | 28 |
| Example 32b washed | 26 | 50348 | 28 |
| Example 33a not washed | 27 | 55174(21) | 31 |
| Example 33b washed | 27 | 51525 (17) | 29 |
| Example 34a not washed | 28 | 73667 | 41 |
| Example 34b washed | 28 | 52473 (11) | 29 |
| N=2, stdev less than 10% unless otherwise noted in parentheses. The matrix of Example 14 had a background signal of 84 RLUs, the matrix of Example 24 was 233 RLUs, the matrix of Example 25 was 90 RLUs, the matrix of Example 26 was 247 RLUs, the matrix of Example 27 was 149 RLUs, and the matrix of Example 28 was 207 RLUs. | | | |

Nonwoven fibrous porous matrices were tested on different days and the ATP signal from the original sample was different on each day. The variation in tests indicates matrix interference. The improvement in ATP signal after washing indicates the decrease in carryover of inhibitory substances, which allows the ATP assay to be used for rapid monitoring of produced water samples.

*Testing of nonwoven fibrous porous matrices with hydroxyapatite for rapid microbial monitoring of E. coli and S. aureus*

**Example 35:** A single colony from a streak culture *of E. coli* (ATCC 51813, a Gram negative organism) was inoculated into 10 ml of TSB (Tryptic Soy Broth, 3% by weight from Difco) incubated overnight for about 20 hours at 37°C. The resulting bacterial stock contained about $1 \times 10^9$ cfus/ml. That stock was serially diluted in DI water to make a working stock of $1 \times 10^5$ cfus/ml.

14 mm disks of nonwoven fibrous porous matrix of Example 24 were die punched and inserted into 13 mm filter holders (SWINNEX holders obtained from Millipore). One ml of the above working stock was filtered through the disk using a 1 cc syringe. The filtrate was discarded. The disk was removed from the holder and placed in a cuvette. A volume of 145 microliters of the CLEAN-TRACE Water-Plus Total ATP extractant. The cuvette was vortexed for 10 seconds. A volume of 450 microliters of the CLEAN-TRACE Water-Plus Total ATP enzyme was added, then mixed for 10 seconds. The cuvette was connected to the adaptor and read in the NG luminometer. Disks through which 1 ml unspiked DI water was filtered were also tested for background ATP signal. This background signal was subtracted from the test results. A one hundred microliter volume from a $1 \times 10^6$ cfus/ml stock was tested for ATP signal. This was the "100% control". Capture efficiency was calculated using the formula of Example 10 above. The matrices of Examples 27 and 28 were tested the same as Example 24. The results for Examples 24, 27, and 28 are shown in Table 14 below.

**Table 14. *E. coli* bacterial capture efficiency of ATP in nonwoven fibrous porous matrices containing metal phosphate**

| Matrix | Example 24 | Example 27 | Example 28 |
|---|---|---|---|
| ATP signal in RLUs in test disks | 4092 | 3505 | 4568 |
| 100 % Control (ATP signal in RLUs) | 13002 | 13002 | 13002 |
| % Capture Efficiency | 31 | 27 | 35 |
| N=2, std deviation under 10% unless specified in parentheses. The matrix of Example 24 had a background value of 395 RLUs, while the matrix of Example 27 had a background value of 278 RLUs, and the matrix of Example 28 had a background of 212 RLUs. | | | |

A single colony from a streak culture of S *aureus* (ATCC 6538, a Gram positive organism) was inoculated into 10 ml of TSB (Tryptic Soy Broth, 3% by weight from Difco) incubated overnight for about 20 hours at 37°C. The resulting bacterial stock contained about $1 \times 10^9$ cfus/ml. That stock was serially diluted in DI water to make a working stock of $1 \times 10^5$ cfus/ml. 14 mm disks from the nonwoven fibrous porous matrices of Examples 24, 27, and 28 were tested as described above. The results are shown in Table 15 below.

**Table 15. *S. aureus* bacterial capture efficiency of ATP in nonwoven fibrous porous matrices containing metal phosphate**

| Matrix | Example 24 | Example 27 | Example 28 |
|---|---|---|---|
| ATP signal in RLUs in test disks | 1003 | 514 | 2050 |
| 100 % Control (ATP signal in RLUs | 4136 | 4136 | 4136 |
| % Capture Efficiency | 24 (36) | 12 (23) | 50 (26) |
| N=2, std deviation under 10% unless specified in parentheses. The matrix of Example 24 had a background value of 395 RLUs, while the matrix of Example 27 had a background value of 278 RLUs, and the matrix of Example 28 had a background of 212 RLUs. | | | |

*Preparation of nonwoven fibrous porous matrices containing metal silicate*

**Matrix of Comparative Example 36:** A fiber premix was prepared by mixing various amounts of Fiber 1, Fiber 2, Fiber 3, and Fiber 4 as shown in Table 16 below. The nonwoven fibrous porous matrix was prepared according to the procedure described above for Examples 1-3. The thickness of the resulting nonwoven fibrous porous matrix was between 0.8 and 1.0 mm.

**Table 16: Composition of Comparative Example 36**

| Material (in grams) | Example 36 |
|---|---|
| Fiber 1 | 11.00 |
| Fiber 2 | 3.00 |
| Fiber 3 | 2.25 |
| Fiber 4 | 1.75 |
| CM-111 | 5.00 |

*Testing of nonwoven fibrous porous matrix with calcined metal silicate for rapid microbial monitoring in beer*

ATP bioluminescence testing has been employed as a method of beer line cleaning verification, which is based on testing of final rinse water after cleaning has been completed. Samples of rinse water are taken from the dispense

side of the line and are then tested using ATP bioluminescence testing. While the test is valuable in terms of cleaning verification, the ability to directly test beer or product samples in the beer line would also bring advantages.

As well as being able to assess the quality of the beer itself, direct testing of the beer product could indirectly indicate the hygienic status of the beer lines and indicate if the beer lines required cleaning. As cleaning lines involves the purging of beer, leading to significant product loss, the ability to clean only when required could potentially result in reduced financial loss where cleaning is only carried out when necessary. Further, the ATP bioluminescence tests use microliter volume samples of rinse water, whereas a filtration test could enable sampling of 50 - 100 ml or more of a final product, which could increase the test sensitivity.

**Example 37:** Two beer samples were obtained from a beer production line outlet. A 50 ml volume from each sample was filtered through a 0.45 micron membrane (a 50 ml tube top filter from Corning obtained from VWR) to remove innate microbial contaminants. A one hundred microliter volume from each sample was added to a 1.5 ml microfuge tube and mixed with 110 microliters of the CLEAN-TRACE Surface ATP extractant and vortexed for 10 seconds. A volume of 250 microliters of the CLEAN-TRACE Surface ATP enzyme was added, then mixed for 10 seconds. The contents were mixed by vortexing for 5 seconds at about 3200 rpm on a vortex mixer (VWR Fixed Speed vortex mixer; VWR, West Chester PA). The ATP signal was determined by measuring the relative light units (RLUs) for a minute at 10 second intervals using a bench-top luminometer (20/20n single tube luminometer from Turner Biosystems, Sunnyvale, CA). Luminescence values were obtained from the luminometer using 20/20n SIS software that was provided with the luminometer. This is the "Unspiked Beer control".

A streak culture of S. *cerevisiae* (ATCC 201390) from a YPD agar plate, incubated at 30°C, was used to make a 0.5 McFarland standard in 3 ml of deionized water using a DENSICHEK densitometer from bioMerieux, Inc., Durham NC. The resulting yeast stock, containing approximately $10^8$ cfu/ml, was diluted serially in filtered beer to obtain a yeast suspension containing $10^5$ cfu/ml. A 1:10 dilution of the suspension was inoculated into 50 ml of beer to provide $10^4$ cfu/ml (total of approximately $5 \times 10^4$ CFUs in 5 ml). The spiked beer was delivered to the SWINNEX filter holder containing a 14 mm nonwoven fibrous porous matrix die-punched from Example 36 using a 10 cc syringe. After the entire 5 mL sample passed through the matrix, the filter holder was disassembled and the disk was transferred, using surface sterilized forceps, to an empty sterile 1.5 ml microfuge tube). The ATP signal on the disk was read as described above. This is the signal for the "Concentrated Sample".

A 100 microliter from the $5 \times 10^5$ cfu/mL stock was used to generate the "100% Control". Disks through which 5 ml filtered beer was processed were tested for background ATP signal similarly (Disk background). A 100 microliter volume from the spiked beer stock was used to generate the "Spiked Beer Control". Nonwoven fibrous porous matrices were tested in duplicates. The % ATP signal was calculated using the formula below:

$$\% \text{ ATP signal} = (\text{RLUs from Concentrated Sample/RLUs from 100\% Control Beer}) \times 100$$

The same procedure for *S. cerevisiae* was performed for both beer samples. Results are shown in Table 17 below.

**Table 17. S. *aureus* bacterial capture efficiency of ATP in nonwoven fibrous porous matrices containing metal silicate**

| Sample | ATP Signal (RLUs) | % ATP |
|---|---|---|
| Unspiked Filtered Beer-1 | 47437 | N/A |
| 100% Control Beer-1 | 351504 | 100% |
| Spiked Beer-1 control | 45808 | 13% |
| Example 36 Beer-1 | 192031 | 55% |
| Example 36 Background Beer-1 | 32659 | N/A |
| Unspiked Filtered Beer-2 | 41664 | N/A |
| 100% Control Beer-2 | 340974 | 100% |
| Spiked Beer-2 control | 43363 | 12% |
| Example 36 Beer-2 | 116579 (20) | 34% |

(continued)

| Sample | ATP Signal (RLUs) | % ATP |
|---|---|---|
| Example 36 background Beer-2 | 31065 | N/A |
| N=2, stdev less than 10% unless otherwise noted in parentheses. Since the nonwoven fibrous porous matrix background signal was not more than the background ATP signal of the beer matrix, it was not subtracted from the test signal. | | |

A 10 fold higher signal was observed with the sample passed through the matrix of Example 36 than with the spiked beer controls, which was very close to the signal of the unpsiked beer sample.

**Examples 38-40:** Making nonwoven fibrous porous matrices with calcined magnesium silicate

Three fiber premixes were prepared by mixing various amounts of Fiber 1, Fiber 2, Fiber 4, Fiber 5 and Fiber 6 as shown in Table 18 below.

For Example 38, the fibers were added to 3 liters of cold deionized water in a 4 L blender (available from VWR, Radnor, PA, under the trade designation "WARING COMMERCIAL HEAVY DUTY BLENDER, MODEL 37BL84") and blended at low speed for 30 seconds. The mixture was examined for uniform dispersion of the fibers without nits or clumps. The additive particles, CM-111, were added with an additional liter of deionized water and mixed at low speed for 15 seconds.

For Examples 39 and 40, Fiber 5 was blended in 3 liters of cold deionized water for 30 seconds at medium speed. All other fibers were added and blended for 30 seconds at low speed. The additive particles, CM-111, were added with an additional liter of deionized water and mixed at low speed for 15 seconds.

A nonwoven fibrous porous felt was prepared using a pad maker apparatus (obtained from Williams Apparatus, Watertown, NY, under the trade designation "TAPPI") that had a box measuring about 30 centimeters (12 inches) square and 30 centimeters (12 inches) high with a fine mesh screen at the bottom and a drain valve. On the screen ~ a 14 inch (36 cm) x 12 inch (30 cm) piece of a polyethylene spunbond (PET Lutradur 7240 obtained from Fiberweb, Cincinnati, OH) was laid as scrim on the screen. The box was filled with tap water up to a height of about 1 centimeter above the screen. Each fiber and additive mixture was poured into the box and the valve was opened immediately which created a vacuum that pulled the water out of the box.

The fibrous nonwoven felts were transferred from the apparatus onto a 20 centimeter square sheet of blotter paper (96-pound white paper, obtained from Anchor Paper, St. Paul, MN). Each felt was sandwiched between 2 to 4 layers of blotter paper, to blot excess water. The pressed felt of Example 38 was then transferred onto a fresh sheet of blotter paper and placed in an oven (HERATHERM oven series OMS100 obtained from Thermo Fisher Scientific, Waltham, MA) set at 130°C for about 3 hours to remove residual water and to form a nonwoven fibrous porous matrix. The felts of Examples 39 and 40 were dried at 125°C for about 3 hours to remove residual water and to form a nonwoven fibrous porous matrix. The resulting fibrous porous matrices of Examples 38 and 40 were approximately 1.30 to 1.40 millimeters thick. The fibrous porous matrix of Example 39 was 1.45-1.55 millimeters thick.

**Table 18: Compositions of Reference Examples 38-40**

| Material (grams) | Example 38 | Example 39 | Example 40 |
|---|---|---|---|
| Fiber 1 | 11.00 | 7.74 | 7.70 |
| Fiber 2 | 3.00 | 3.06 | 3.0 |
| Fiber 4 | 1.75 | 1.78 | 1.75 |
| Fiber 5 | 0 | 8.06 | 0 |
| Fiber 6 | 2.25 | 2.28 | 2.30 |
| Fiber 7 | 0 | 0 | 11.78 |
| CM-111 | 10.00 | 10.00 | 10.01 |
| Basis weight (g/m$^2$) | 275.02 | 261.89 | 276.20 |

*Testing of nonwoven fibrous porous matrices with metal silicate for bacterial capture of E. coli*

A single colony from a streak culture *of E. coli* (ATCC 11229, a Gram negative organism) was inoculated into

10 ml of TSB (Tryptic Soy Broth, 3% by weight from Difco) incubated overnight for about 20 hours at 37 degrees C. The resulting bacterial stock contained about $1 \times 10^9$ cfus/ml. That stock was serially diluted in deionized water to make a working stock of $1 \times 10^2$ cfus/ml.

**Reference Examples 41-43:** 14 mm disks of nonwoven fibrous porous matrix of Example 38-40, respectively, were each die punched and inserted into 13 mm filter holders (Swinnex holders obtained from Millipore). Ten ml of the above working stock was filtered through the disk using a 10 cc syringe. After filtration the disks were discarded. The filtrates were collected in sterile 15 ml polypropylene tubes. A one ml volume of each filtrate was plated on PAC plates and tested for *E coli* capture.

The stock solution was also plated on PAC. This was the "100% control". Plate counts were measured per manufacturer's instructions using a PETRIFILM Plate Reader. Capture efficiency was calculated using the formulas below. The results are shown in Table 19.

$$\% \text{ Control} = (\text{CFUs in plated filtrates}) \times 100/\text{CFUs in } 100\% \text{ Control}$$

$$\% \text{ Capture Efficiency} = 100 - \% \text{ Control}$$

**Table 19 Capture of *E. coli***

| Sample | Example 41 | Example 42 | Example 43 |
|---|---|---|---|
| % Capture Efficiency | 71.7(13) | 52.7 (11) | 95.1 |
| n=3, % std deviation less than 10% unless otherwise noted in parentheses. The plated 100% control had an average of 163.5 cfus/ml (26% stdev, total 1635 cfus in 10 ml). | | | |

*Testing of nonwoven fibrous porous matrices with metal silicate for bacterial capture of S. aureus*

A single colony from a streak culture of S. *aureus* (ATCC 6538, a Gram positive organism) was inoculated into 10 ml of TSB (Tryptic Soy Broth, 3% by weight from Difco) incubated overnight for about 20 hours at 37 degrees C. The resulting bacterial stock contained about $1 \times 10^9$ cfus/ml. That stock was serially diluted in deionized water to make a working stock of $1 \times 10^2$ cfus/ml.

**Reference Examples 44-46:** 14 mm disks of nonwoven fibrous porous matrix of each of Examples 38-40, respectively, were die punched and inserted into 13 mm filter holders (Swinnex holders obtained from Millipore). Ten ml of above working stock was filtered through the disk using a 10 cc syringe. After filtration the disks were discarded. The filtrates were collected in sterile 15 ml polypropylene tubes. A one ml volume of each filtrate was plated on PAC plates. Each disk was tested for *S aureus* capture.

The stock solution was also plated on PAC. This was the "100% control". Plate counts were measured per manufacturer's instructions using a PETRIFILM Plate Reader. Capture efficiency was calculated using the formulas below. The results are shown in Table 20.

$$\% \text{ Control} = (\text{CFUs in plated filtrates}) \times 100/\text{CFUs in } 100\% \text{ Control}$$

$$\% \text{ Capture Efficiency} = 100 - \% \text{ Control}$$

**Table 20: Capture of S. *aureus***

| Sample | Example 44 | Example 45 | Example 46 |
|---|---|---|---|
| % Capture Efficiency | 99.36 | 100 | 99.36 |
| n=3, % std deviation less than 10% unless otherwise noted in parentheses. The plated 100% control had an average 156 cfus/ml (total 1566 cfus in 10 ml). | | | |

**Examples 47 and 48:** Making thin nonwoven fibrous porous matrices containing calcined magnesium silicate

Two fiber premixes were prepared by mixing various amounts of Fiber 1, Fiber 4, Fiber 5 and Fiber 6 as shown in Table 21 below.

For Example 47, the Fiber 6 was added to 3 liters of cold deionized water in a 4 L blender (available from VWR, Radnor, PA, under the trade designation "WARING COMMERCIAL HEAVY DUTY BLENDER, MODEL 37BL84") and blended at medium speed for 30 seconds. All other fibers were added and blended for additional 1 minute at low speed. The mixture was examined for uniform dispersion of the fibers without nits or clumps. The particle additive, CM-111, was added with an additional liter of deionized water and mixed at low speed for 15 seconds.

A nonwoven fibrous porous felt was prepared using a pad maker apparatus (obtained from Williams Apparatus, Watertown, NY, under the trade designation "TAPPI") that had a box measuring about 30 centimeters (12 inches) square and 30 centimeters (12 inches) high with a fine mesh screen at the bottom and a drain valve. On the screen ~ a 14 inch (36 cm) × 12 inch (30 cm) piece of a polyethylene spunbond (PET Lutradur 7240 obtained from Fiberweb, Cincinnati, OH) was laid as scrim on the screen. The box was filled with tap water up to a height of about 1 centimeter above the screen. The fiber and particle additive mixture was poured into the box and the valve was opened immediately which created a vacuum that pulled the water out of the box.

The fibrous nonwoven felt was transferred from the apparatus onto a 20 centimeter square sheet of blotter paper (96-pound white paper, obtained from Anchor Paper, St. Paul, MN). The felt was sandwiched between 2 to 4 layers of blotter paper, to blot excess water. The pressed felt of Example 29 was then transferred onto a fresh sheet of blotter paper and placed in an oven (HERATHERM oven series OMS100 obtained from Thermo Fisher Scientific, Waltham, MA) set at 125°C for about 3 hours to remove residual water and to form a nonwoven fibrous porous matrix. The resulting fibrous porous matrix of Example 47 was approximately 0.8 to 0.90 millimeters thick.

For Example 48, the fiber mixture was prepared as described for Example 29, except as noted, using the amounts shown in Table 16 below. The pad maker apparatus was filled with additional 4 liters of cold deionized water. No spunbond scrim was used on the screen of the apparatus. The fiber and particle additive mixture was poured into the apparatus while opening the valve and draining the water. The fibrous nonwoven felt was removed from the screen by pressing a ~ 14 inch (36 cm) × 12 inch (30 cm) piece of a polyethylene spunbond onto the wet felt and lifting the scrim. The resulting dried nonwoven fibrous porous matrix was approximately 0.8 to 0.90 millimeters thick.

**Table 21**

| Material (grams) | Example 47 | Example 48 |
|---|---|---|
| Fiber 1 | 6.02 | 6.02 |
| Fiber 4 | 1.07 | 1.06 |
| Fiber 5 | 1.30 | 1.32 |
| Fiber 6 | 1.05 | 2.05 |
| CM-111 | 4.03 | 4.08 |
| Basis Weights in $g/m^2$ | 126.05 | 122.17 |

*Testing of nonwoven fibrous porous matrices with metal silicate for bacterial capture of E. coli*

A single colony from a streak culture *of E. coli* (ATCC 11229, a Gram negative organism) was inoculated into 10 ml of TSB (Tryptic Soy Broth, 3% by weight from Difco) incubated overnight for about 20 hours at 37 degrees C. The resulting bacterial stock contained about $1 \times 10^9$ cfus/ml. That stock was serially diluted in deionized water to make working stocks of 10 cfus/ml, $1 \times 10^2$ cfus/ml, and $1 \times 10^3$ cfus/ml.

**Example 49:** 14 mm disks of the nonwoven fibrous porous matrix of Example 47 were die punched and inserted into 13 mm filter holders (SWINNEX holders obtained from Millipore). 10 ml of 10 cfus/ml working stock was filtered through each disk using a 10 cc syringe. After filtration the disks were discarded. The filtrate was collected in sterile 15 ml polypropylene tubes. A one ml volume of the filtrate was plated on PAC plates.

**Example 50:** 14 mm disks of the nonwoven fibrous porous matrix of Example 47 were die punched and inserted into 13 mm filter holders (SWINNEX holders obtained from Millipore) and tested for *E. coli* capture using 10 ml of $1\times10^2$ cfus/ml working stock according to the same procedure as Example 49.

**Example 51:** 14 mm disks of the nonwoven fibrous porous matrix of Example 47 were die punched and inserted into 13 mm filter holders (SWINNEX holders obtained from Millipore) and tested for *E. coli* capture using 10 ml of $1\times10^3$ cfus/ml working stock according to the same procedure as Example 49.

**Examples 52 to 54:** 14 mm disks of the nonwoven fibrous porous matrix of Example 48 were tested according to the same procedures as Examples 49, 50, and 51, to generate Examples 52, 53 and 54, respectively.

The *E. coli* stock solutions were also plated on PAC. These were the "100% control" samples. Plate counts were measured per the manufacturer's instructions using a PETRIFILM Plate Reader. Capture efficiency was calculated using the formulas below. The results are shown in Table 22.

$$\% \text{ Control} = (\text{CFUs in plated filtrates}) \times 100/\text{CFUs in } 100\% \text{ Control}$$

$$\% \text{ Capture Efficiency} = 100 - \% \text{ Control}$$

**Table 22. Capture of *E. coli***

| Sample | Example 49 | Example 50 | Example 51 | Example 52 | Example 53 | Example 54 |
|---|---|---|---|---|---|---|
| % Capture Efficiency | 70.7 (48) | 53.4(11) | 67.1 (13) | 78.8 (26) | 71.3 | 82.8 |
| n=3, % std deviation < 10% unless indicated in parenthesis. The plated 100% control had total 205 cfus/ml (24% stdev), 1895 cfus and 18450 cfus. | | | | | | |

*Testing of nonwoven fibrous porous matrices with metal silicate for bacterial capture of S. aureus*

A single colony from a streak culture of S. *aureus* (ATCC 6538, a Gram positive organism) was inoculated into 10 ml of TSB (Tryptic Soy Broth, 3% by weight from Difco) incubated overnight for about 20 hours at 37 degrees C. The resulting bacterial stock contained about $1 \times 10^9$ cfus/ml. That stock was serially diluted in deionized water to make working stocks of 10 cfus/ml, $1 \times 10^2$ cfus/ml, and $1 \times 10^3$ cfus/ml.

**Example 55:** 14 mm disks of the nonwoven fibrous porous matrix of Example 47 were die punched and inserted into 13 mm filter holders (SWINNEX holders obtained from Millipore). 10 ml of 10 cfus/ml working stock was filtered through each disk using a 10 cc syringe. After filtration the disks were discarded. The filtrate was collected in sterile 15 ml polypropylene tubes. A one ml volume of the filtrate was plated on PAC plates.

**Example 56:** 14 mm disks of the nonwoven fibrous porous matrix of Example 47 were die punched and inserted into 13 mm filter holders (Swinnex holders obtained from Millipore) and tested for S. *aureus* capture using 10 ml of $1 \times 10^2$ cfus/ml working stock according to the same procedure as Example 55.

**Example 57:** 14 mm disks of the nonwoven fibrous porous matrix of Example 47 were die punched and inserted into 13 mm filter holders (Swinnex holders obtained from Millipore) and tested for S. *aureus* capture using 10 ml of $1 \times 10^3$ cfus/ml working stock according to the same procedure as Example 55.

**Examples 58 to 60:** 14 mm disks of the nonwoven fibrous porous matrix of Example 48 were tested according to the same procedures as Examples 55, 56, and 57, to generate Examples 58, 59, and 60, respectively.

The S. *aureus* stock solution was also plated on PAC. These were the "100% control" samples. Plate counts were measured per the manufacturer's instructions using a PETRIFILM Plate Reader. Capture efficiency was calculated using the formulas below. The results are shown in Table 23.

$$\% \text{ Control} = (\text{CFUs in plated filtrates}) \times 100/\text{CFUs in } 100\% \text{ Control}$$

$$\% \text{ Capture Efficiency} = 100 - \% \text{ Control}$$

**Table 23. Capture of *S. aureus***

| Sample | Example 55 | Example 56 | Example 57 | Example 58 | Example 59 | Example 60 |
|---|---|---|---|---|---|---|
| % Capture Efficiency | 96.6 | 95.2 | 96.5 | 98.3 | 95.7 | 96.5 |
| n=3, % std deviation < 10 % unless indicated in parentheses. The plated 100% control had total 195 cfus/ml (40% stdev), 1380 cfus (12% stdev) and 15950 cfus. | | | | | | |

While the specification has described in detail certain exemplary embodiments, it will be appreciated that those skilled in the art, upon attaining an understanding of the foregoing, may readily conceive of alterations to, variations of, and equivalents to these embodiments. Various exemplary embodiments have been described. These and other embodiments are within the scope of the following claims.

## Claims

1. A nonwoven article comprising a) a fibrous porous matrix and b) a plurality of concentration agent particles enmeshed in the fibrous porous matrix, the fibrous porous matrix consisting essentially of inorganic fibers and polymeric fibers, wherein the inorganic fibers comprise glass fibers, wherein the polymeric fibers comprise fibrillated polyethylene fibers and bi-component fibers, wherein the fibrous porous matrix is free of polyamide fibers, wherein the fibrous porous matrix is a nonwoven fibrous layer comprising inorganic fibers and uncrimped polymeric fibers, and wherein the concentration agent particles comprise amorphous metal silicates, guanidine-functionalized metal silicates, diatomaceous earth, surface-modified diatomaceous earth, gamma-FeO(OH), metal carbonates, metal phosphates, silica, perlite, guanidine-functionalized perlite, guanidine-functionalized diatomaceous earth, or a combination thereof.

2. The nonwoven article of claim 1, wherein the nonwoven article has a higher percent adenosine triphosadenine recovery than a comparative nonwoven article comprising the same fibers except further comprising nylon fibers in ATP bioluminescence assay.

3. The nonwoven article of any of claims 1 to 2, wherein the inorganic fibers and polymeric fibers comprise an average length of less than 50 millimeters.

4. The nonwoven article of any of claims 1 to 3, wherein the nonwoven article comprises 5 to 60 weight percent concentration agent particles based on a total dried weight of the nonwoven article and 40 to 95 weight percent fibrous porous matrix based on the total dried weight of the nonwoven article.

5. The nonwoven article of any of claims 1 to 4, wherein the fibrous porous matrix has a thickness of between 0.15 millimeters and 2 millimeter.

6. The nonwoven article of any of claims 1 to 5, wherein the concentration agent particles comprise amorphous metal silicates.

7. A method of detecting microorganisms or target cellular analytes in a fluid sample, the method comprising:

   a) providing a nonwoven article according to any of claims 1 to 6;
   b) providing a fluid sample suspected of containing at least one microorganism strain or target cellular analyte;
   c) contacting the fluid sample with the nonwoven article such that at least a portion of the at least one microorganism strain or target cellular analyte is bound to the nonwoven article; and
   d) detecting the presence of the at least one bound microorganism strain or bound target cellular analyte.

8. The method of claim 7, further comprising placing the at least one microorganism strain- or target cellular analyte-bound nonwoven article in contact with at least one detection reagent before detecting the presence of the at least one bound microorganism strain or bound target cellular analyte.

9. The method of claim 7 or claim 8, wherein the detecting comprises a bioluminescence method.

10. The method of any of claims 7 to 9, further comprising contacting the at least one bound microorganism strain with a lysis agent.

11. The method of any of claims 7 to 10, further comprising washing the at least one microorganism strain- or target cellular analyte-bound nonwoven article prior to the placing the at least one microorganism strain- or target cellular analyte-bound nonwoven article in contact with at least one detection reagent.

12. The method of any of claims 9 to 11, wherein the fluid sample comprises a rinsate from a lumened or cannulated device and the contacting occurs in an integrated assembly in which the lumened or cannulated device is in fluid

communication with the nonwoven article.

## Patentansprüche

1. Ein Vliesartikel, umfassend a) eine faserige poröse Matrix und b) eine Vielzahl von Konzentriermittelteilchen, die in der faserigen porösen Matrix ineinandergreifen, wobei die faserige poröse Matrix im Wesentlichen aus anorganischen Fasern und Polymerfasern besteht, wobei die anorganischen Fasern Glasfasern umfassen, wobei die Polymerfasern fibrillierte Polyethylenfasern und Bikomponentenfasern umfassen, wobei die faserige poröse Matrix frei von Polyamidfasern ist, wobei die faserige poröse Matrix eine Vliesfaserschicht ist, die anorganische Fasern und ungekräuselte Polymerfasern umfasst, und wobei die Konzentriermittelpartikel amorphe Metallsilikate, guanidinfunktionalisierte Metallsilikate, Kieselgur, oberflächenmodifizierten Kieselgur, gamma-FeO(OH), Metallcarbonate, Metallphosphate, Siliziumdioxid, Perlit, guanidinfunktionalisiertes Perlit, guanidinfunktionalisierten Kieselgur oder eine Kombination davon umfassen.

2. Der Vliesartikel nach Anspruch 1, wobei der Vliesartikel eine höhere prozentuale Adenosin-Triphosadenin-Rückgewinnung als ein Vergleichsvliesartikel aufweist, der die gleichen Fasern umfasst, außer ferner umfassend Nylonfasern in ATP-Biolumineszenz-Assay.

3. Der Vliesartikel nach einem der Ansprüche 1 bis 2, wobei die anorganischen Fasern und Polymerfasern eine durchschnittliche Länge von weniger als 50 Millimetern umfassen.

4. Der Vliesartikel nach einem der Ansprüche 1 bis 3, wobei der Vliesartikel 5 bis 60 Gew.-% Konzentriermittelpartikel, bezogen auf ein gesamtes getrocknetes Gewicht des Vliesartikels, und 40 bis 95 Gew.-% faserige poröse Matrix, bezogen auf das gesamte getrocknete Gewicht des Vliesartikels, umfasst.

5. Der Vliesartikel nach einem der Ansprüche 1 bis 4, wobei die faserige poröse Matrix eine Dicke zwischen 0,15 Millimeter und 2 Millimeter aufweist.

6. Der Vliesartikel nach einem der Ansprüche 1 bis 5, wobei die Konzentriermittelpartikel amorphe Metallsilikate umfassen.

7. Ein Verfahren zum Detektieren von Mikroorganismen oder Zielzellanalyten in einer Fluidprobe, wobei das Verfahren umfasst:

   a) Bereitstellen eines Vliesartikels nach einem der Ansprüche 1 bis 6;
   b) Bereitstellen einer Fluidprobe, von der angenommen wird, dass sie mindestens einen Mikroorganismenstamm oder Zielzellanalyten enthält;
   c) Inkontaktbringen der Fluidprobe mit dem Vliesartikel, so dass mindestens ein Abschnitt des mindestens einen Mikroorganismenstamms oder Zielzellanalyten an den Vliesartikel gebunden ist; und
   d) Erfassen des Vorhandenseins des mindestens einen gebundenen Mikroorganismenstamms oder gebundenen Zielzellanalyten.

8. Das Verfahren nach Anspruch 7, ferner umfassend das Platzieren des mindestens einen mikroorganismenstamm- oder zielzellanalytgebundenen Vliesartikels in Kontakt mit mindestens einem Nachweisreagenz vor dem Erfassen des Vorhandenseins des mindestens einen gebundenen Mikroorganismenstamms oder gebundenen Zielzellanalyten.

9. Das Verfahren nach Anspruch 7 oder Anspruch 8, wobei das Erfassen ein Biolumineszenzverfahren umfasst.

10. Das Verfahren nach einem der Ansprüche 7 bis 9, ferner umfassend das Inkontaktbringen des mindestens einen gebundenen Mikroorganismenstamms mit einem Lysemittel.

11. Verfahren nach einem der Ansprüche 7 bis 10, ferner umfassend das Waschen des mindestens einen mikroorganismenstamm- oder zielzellanalytgebundenen Vliesartikels vor dem Platzieren des mindestens einen mikroorganismenstamm- oder zielzellanalytgebundenen Vliesartikels in Kontakt mit mindestens einem Nachweisreagenz.

12. Das Verfahren nach einem der Ansprüche 9 bis 11, wobei die Fluidprobe ein Rinsat aus einer lumigen oder kanülierten

Vorrichtung umfasst und das Inkontaktbringen in einer integrierten Baugruppe erfolgt, in der die lumige oder kanülierte Vorrichtung in Fluidverbindung mit dem Vliesartikel ist.

## Revendications

1. Article non tissé comprenant a) une matrice poreuse fibreuse et b) une pluralité de particules d'agent de concentration enchevêtrées dans la matrice poreuse fibreuse, la matrice poreuse fibreuse constituée sensiblement de fibres inorganiques et de fibres polymères, dans lequel les fibres inorganiques comprennent des fibres de verre, dans lequel les fibres polymères comprennent des fibres de polyéthylène fibrillaires et des fibres bicomposantes, dans lequel la matrice poreuse fibreuse est exempte de fibres de polyamide, dans lequel la matrice poreuse fibreuse est une couche fibreuse non tissée comprenant des fibres inorganiques et des fibres polymères non crêpées, et dans lequel les particules d'agent de concentration comprennent des silicates de métal amorphes, des silicates de métal à fonctionnalisation guanidine, de la terre à diatomées, de la terre à diatomées modifiée en surface, du gamma-FeO(OH), des carbonates de métal, des phosphates de métal, de la silice, de la perlite, de la perlite à fonctionnalisation guanidine, de la terre à diatomées à fonctionnalisation guanidine, ou une combinaison de ceux-ci.

2. Article non tissé selon la revendication 1, dans lequel l'article non tissé a un pourcentage plus élevé de récupération d'adénosine triphosadénine qu'un article non tissé comparatif comprenant les mêmes fibres si ce n'est qu'elle comprend en outre des fibres de nylon dans un dosage par bioluminescence d'ATP.

3. Article non tissé selon l'une quelconque des revendications 1 à 2, dans lequel les fibres inorganiques et les fibres polymères comprennent une longueur moyenne inférieure à 50 millimètres.

4. Article non tissé selon l'une quelconque des revendications 1 à 3, dans lequel l'article non tissé comprend 5 à 60 pour cent en poids de particules d'agent de concentration sur la base d'un poids séché total de l'article non tissé et 40 à 95 pour cent en poids de matrice poreuse fibreuse sur la base du poids séché total de l'article non tissé.

5. Article non tissé selon l'une quelconque des revendications 1 à 4, dans lequel la matrice poreuse fibreuse a une épaisseur comprise entre 0,15 millimètre et 2 millimètres.

6. Article non tissé selon l'une quelconque des revendications 1 à 5, dans lequel les particules d'agent de concentration comprennent des silicates de métal amorphes.

7. Procédé de détection de micro-organismes ou d'analytes cellulaires cibles dans un échantillon de fluide, le procédé comprenant :

   a) la fourniture d'un article non tissé selon l'une quelconque des revendications 1 à 6 ;
   b) la fourniture d'un échantillon de fluide soupçonné de contenir au moins une souche de micro-organisme ou un analyte cellulaire cible ;
   c) la mise en contact de l'échantillon de fluide avec l'article non tissé de telle sorte qu'au moins une partie de l'au moins une souche de micro-organisme ou de l'analyte cellulaire cible est liée à l'article non tissé ; et
   d) la détection de la présence de l'au moins une souche de micro-organisme liée ou de l'analyte cellulaire cible lié.

8. Procédé selon la revendication 7, comprenant en outre la mise en place de l'au moins un article non tissé lié à une souche de micro-organisme ou à un analyte cellulaire cible en contact avec au moins un réactif de détection avant détection de la présence de l'au moins une souche de micro-organisme liée ou analyte cellulaire cible lié.

9. Procédé selon la revendication 7 ou la revendication 8, dans lequel la détection comprend un procédé par bioluminescence.

10. Procédé selon l'une quelconque des revendications 7 à 9, comprenant en outre la mise en contact de l'au moins une souche de micro-organisme liée avec un agent de lyse.

11. Procédé selon l'une quelconque des revendications 7 à 10, comprenant en outre le lavage de l'article non tissé lié à au moins une souche de micro-organisme ou à un analyte cellulaire cible avant la mise en place de l'article non tissé lié à au moins une souche de micro-organisme ou à un analyte cellulaire cible en contact avec au moins un réactif de détection.

**12.** Procédé selon l'une quelconque des revendications 9 à 11, dans lequel l'échantillon de fluide comprend un rinçage provenant d'un dispositif à lumière ou à canule et la mise en contact se produit dans un ensemble intégré dans lequel le dispositif à lumière ou à canule est en communication fluidique avec l'article non tissé.

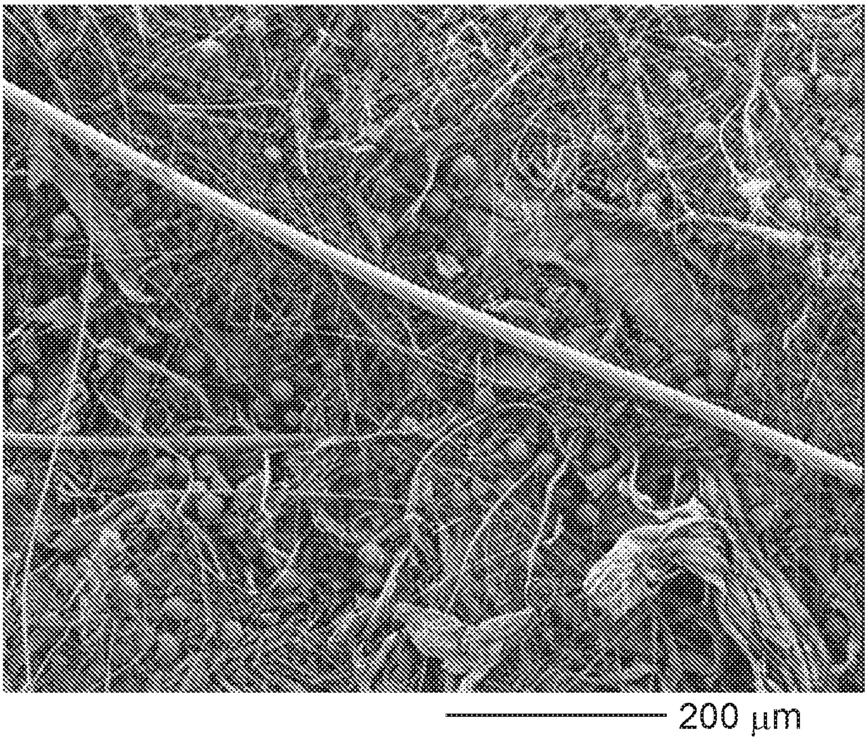


FIG. 1

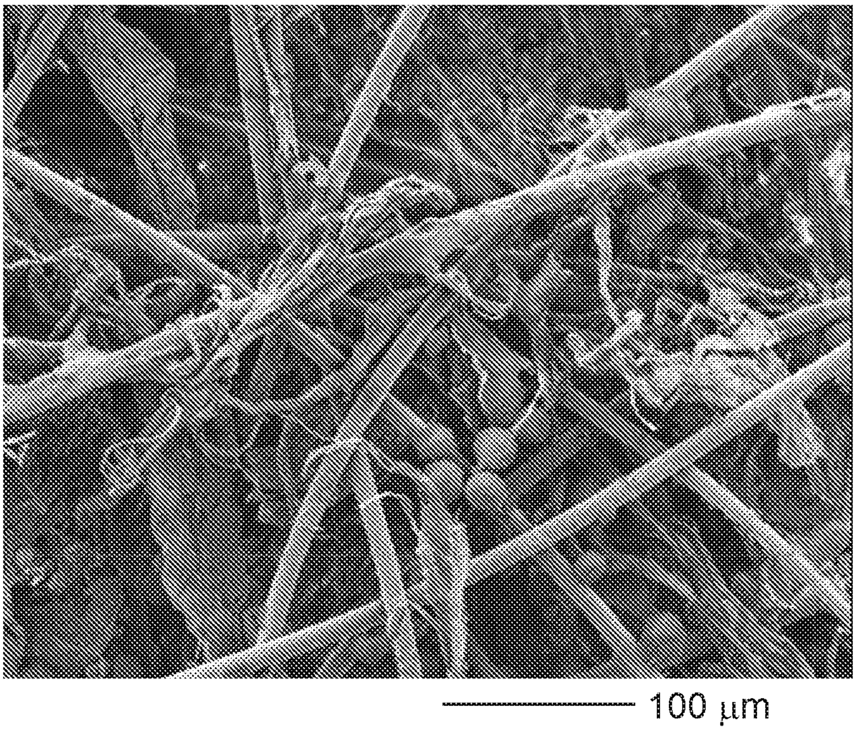


FIG. 2

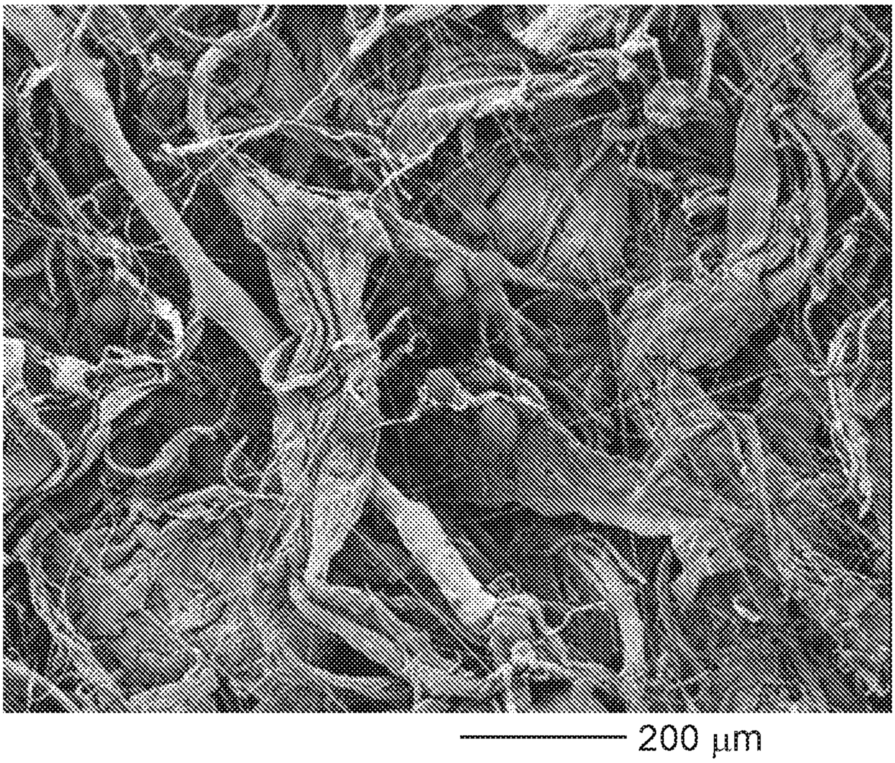


FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014099233 A
- WO 2012078374 A
- WO 2012078426 A
- WO 2010078284 A
- WO 2010078404 A
- WO 2009067518 A
- WO 9306924 A
- US 4118531 A, Hauser
- US 5597645 A, Pike
- CA 2612854, Sommer
- US 6045913 A, Castle
- US 2014040861 W, Kshirsagar
- WO 2009046191 A, Kshirsagar
- WO 2009046183 A, Kshirsagar
- US 4729846 A, Matsui
- US 2018051313 A
- US 7422868 B, Fan
- US 62135303